# EUROPEAN PATENT APPLICATION

(11) **EP 3 466 459 A1**
(43) Date of publication of application: **10.04.2019**
(21) Application number: 18198339.6
(22) Date of filing: 02.10.2018
(51) Int. Cl.: A61M 1/16, A61M 1/28

(54) **PERITONEAL DIALYSATE PREPARATION AND SENSOR SYSTEM**

(30) Priority: 03.10.2017 US 201715723676
(71) Applicant: Medtronic, Inc., Minneapolis, Minnesota 55432 (US)
(72) Inventor: GERBER, Martin T., Maple Grove, MN Minnesota 55369 (US); HOBOT, Christopher M., Rogers, MN Minnesota 55374 (US); Lura, David B., Maple Grove,, MN Minnesota 55311 (US); MANDA, VenKatesh, Stillwater, MN Minnesota 55082 (US); Meyer, Thomas, Stillwater, MN Minnesota 55082 (US)
(74) Representative: Maschio, Antonio

(57) **Abstract**

The invention relates to devices, systems, and methods for generating a peritoneal dialysate having specified concentrations of one or more solutes. The devices, systems and methods use conductivity sensors, flow meters, and composition sensors to control addition of osmotic agents and ion concentrates into a peritoneal dialysate generation flow path.

## Description

### CROSS REFERENCE TO RELATED APPLICATIONS

This application is a continuation in part of U.S. Patent Application No. 15/478,569 filed April 4, 2017, which claims benefit of and priority to U.S. Provisional Application No. 62/318,173 filed April 4, 2016, and the disclosures of each of the above-identified applications are hereby incorporated by reference in their entirety.

### FIELD OF THE INVENTION

The invention relates to devices, systems, and methods for generating a peritoneal dialysate having specified concentrations of one or more solutes. The devices, systems and methods use conductivity sensors, flow meters, and composition sensors to control addition of osmotic agents and ion concentrates into a peritoneal dialysate generation flow path.

### BACKGROUND

Peritoneal Dialysis (PD), including Automated Peritoneal Dialysis (APD) and Continuous Ambulatory Peritoneal Dialysis (CAPD) can be performed at a clinic or in a home-setting either by a patient alone or with a care-giver. PD differs from Hemodialysis (HD) in that blood is not removed from the body and passed through a dialyzer, but rather a catheter is placed in the peritoneal cavity and dialysate introduced directly into the peritoneal cavity. Blood is cleaned inside the patient using the patient's own peritoneum as a type of dialysis membrane. However, because fluid is directly introduced into a human body, the fluid used for peritoneal dialysate is generally required to be free of biological and chemical contaminants. The peritoneal dialysate should also contain specified concentrations of solutes and cations for biocompatibility and for performing membrane exchange.

Known systems and methods cannot generate a peritoneal dialysate having specific and customizable solute concentrations for infusion into a patient. The known systems and methods also cannot modify the composition of the peritoneal dialysate based on a specified dialysate prescription. Importantly, the known systems and methods use premade dialysate formulations that cannot be altered based on the specific needs of individual patients.

As such, there is a need for systems and methods that can generate peritoneal dialysate having specific concentrations of solutes. The systems and methods should include sensors for measuring the solute concentrations of the generated dialysate and for ensuring the generated peritoneal dialysate matches a dialysate prescription.

### SUMMARY OF THE INVENTION

The first aspect of the invention relates to a dialysate preparation system for use in peritoneal dialysis. In any embodiment, the dialysate preparation system can comprise a first fluid line fluidly connected to a water purification module; at least one ion concentrate source fluidly connected to the first fluid line through a first infusate line; the first infusate line having a first concentrate pump; one or more osmotic agent sources fluidly connected to the first fluid line through one or more secondary infusate lines; the secondary infusate lines comprising a secondary concentrate pump forming part of the one or more secondary infusates lines; wherein at least one or more conductivity sensors are positioned in the first fluid line upstream of the first infusate line; at least one or more second conductivity sensors are positioned in the first fluid line downstream of the first infusate line and upstream of the secondary infusate lines; and at least one composition sensor positioned in the first fluid line downstream of the one or more secondary infusate lines; the first fluid line fluidly connectable to an integrated cycler.

In any embodiment, the system can comprise at least one secondary composition sensor positioned in the one or more secondary infusate lines.

In any embodiment, the system can comprise a control system in communication with the composition sensor and secondary composition sensor, the control system measuring an osmotic agent concentration at the composition sensor and secondary composition sensor.

In any embodiment, the control system can control an osmotic agent flow rate based on the composition sensor and secondary composition sensor.

In any embodiment, the system can comprise at least one flow meter in the first fluid line.

In any embodiment, the flow meter can be downstream of the secondary infusate line.

In any embodiment, at least two osmotic agent sources can be fluidly connected to the one or more secondary infusate lines.

In any embodiment, the system can comprise one or more valves fluidly connecting the at least two osmotic agent sources to the secondary infusate lines.

In any embodiment, the system can comprise a control system in communication with the conductivity sensor and secondary conductivity sensor, the control system controlling an ion concentrate flow rate based on the conductivity sensor and secondary conductivity sensor.

In any embodiment, the system can comprise at least one pH sensor in the first fluid line.

In any embodiment, the composition sensor and/or secondary composition sensor can be selected from the group consisting of a refractive index sensor, an enzyme-based sensor, and a pulsed amperometric detection sensor.

In any embodiment, the system can comprise a second fluid line fluidly connecting the second infusate line to a sterilization module.

The features disclosed as being part of the first aspect of the invention can be in the first aspect of the invention, either alone or in combination, or follow a preferred arrangement of one or more of the described elements.

The second aspect of the invention is directed to a method. In any embodiment, the method can comprise the steps of (a) pumping water from a water source through a water purification module into a first fluid line; (b) measuring a first conductivity of fluid in the first fluid line; (c) pumping an ion concentrate from at least one ion concentrate source through a first infusate line into the first fluid line; (d) measuring a second conductivity of the fluid in the first fluid line downstream of the first infusate line; (e) pumping an osmotic agent concentrate from an osmotic agent source through a second infusate line into the first fluid line; and (f) measuring a first osmotic agent concentration in the first fluid line downstream of the second infusate line.

In any embodiment, the method can comprise measuring a second osmotic agent concentration in the second infusate line.

In any embodiment, the method can comprise pumping fluid from the first fluid line into a sterilization module and pumping the fluid from the sterilization module into an integrated cycler.

In any embodiment, the method can comprise receiving a dialysate prescription; and setting an ion concentrate flow rate and an osmotic agent flow rate based on the dialysate prescription.

In any embodiment, the step of setting an ion concentrate flow rate and an osmotic agent flow rate can be performed by a control system in communication with a first concentrate pump in the first infusate line and a second concentrate pump in the second infusate line.

In any embodiment, the controller can set the osmotic agent flow rate based on the first osmotic agent concentration and the dialysate prescription.

In any embodiment, the method can comprise generating an alert if the first osmotic agent concentration is outside of a predetermined range from the dialysate prescription.

In any embodiment, the method can comprise generating an alert if the second conductivity is outside of a predetermined range from the dialysate prescription.

In any embodiment, at least two osmotic agent sources can be fluidly connected to the second infusate line.

In any embodiment, the method can comprise selecting an osmotic agent source from the at least two osmotic agent sources; and pumping the osmotic agent concentrate from the selected osmotic agent source.

In any embodiment, the method can comprise either or both of a) generating the ion concentrate by pumping purified water from a sterilization module into the ion concentrate source; and/or b) generating the osmotic agent concentrate pumping purified water from the sterilization module into the osmotic agent source.

In any embodiment, either or both of a) the step of generating the ion concentrate can comprise agitating the ion concentrate after pumping the purified water into the ion concentrate source, heating the purified water prior to pumping the purified water into the ion concentrate source, or combinations thereof; and/or b) the step of generating the osmotic agent concentrate can comprise agitating the osmotic agent concentrate after pumping the purified water into the osmotic agent source, heating the purified water prior to pumping the purified water into the osmotic agent source, or combinations thereof.

The features disclosed as being part of the second aspect of the invention can be in the second aspect of the invention, either alone or in combination, or follow a preferred arrangement of one or more of the described elements.

### BRIEF DESCRIPTION OF THE DRAWINGS

FIG. 1 shows a peritoneal dialysate generation flow path with an integrated cycler.
FIG. 2 shows a system for adding concentrates to a peritoneal dialysate generation flow path.
FIG. 3 shows an overview of a system for generating and using peritoneal dialysate with a single concentrate source.
FIG. 4 shows an overview of a system for generating and using peritoneal dialysate with multiple concentrate sources.
FIG. 5 shows an alternative peritoneal dialysate generation flow path with an integrated cycler.
FIG. 6 shows a peritoneal dialysate generation flow path with multiple dispensing options.
FIG.'s 7A-D show a peritoneal dialysate generation cabinet with a water reservoir and waste reservoir.
FIG. 8 shows a peritoneal dialysate generation cabinet connected to a faucet and drain.
FIG. 9 shows a peritoneal dialysate generation and delivery system.

### DETAILED DESCRIPTION OF THE INVENTION

Unless defined otherwise, all technical and scientific terms used have the same meaning as commonly understood by one of ordinary skill in the art.

The articles "a" and "an" are used to refer to one or to over one (*i.e.,* to at least one) of the grammatical object of the article. For example, "an element" means one element or over one element.

The terms "agitating" or to "agitate" refer to mixing or otherwise moving a fluid or substance by mechanical means.

The term "communication" refers to an electronic or wireless link between two components.

A "composition sensor" is a device capable of measuring a concentration of one or more solutes in a fluid.

The term "comprising" includes, but is not limited to, whatever follows the word "comprising." Use of the term indicates the listed elements are required or mandatory but that other elements are optional and may be present.

A "concentrate pump" is a pump configured to move fluid between a concentrate source and a flow path.

A "conductivity sensor" is device for measuring the electrical conductance of a solution and/or the ion, such as a sodium ion, concentration of a solution.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "control," "controlling," or "controls" refers to the ability of one component to direct the actions of a second component.

A "control system" can be a combination of components acting together to maintain a system to a desired set of performance specifications. The control system can use processors, memory and computer components configured to interoperate to maintain the desired performance specifications. The control system can also include fluid or gas control components, and solute control components as known within the art to maintain the performance specifications.

The term "dialysate" describes a fluid into or out of which solutes from a fluid to be dialyzed diffuse through a membrane. For example, for peritoneal dialysis, solutes can be diffused through a peritoneal membrane of a patient. Dialysate can differ depending on the type of dialysis to be carried out. For example, dialysate for peritoneal dialysis may include different solutes or different concentrations of solutes than dialysate for hemodialysis.

The term "dialysate preparation system" refers to a set of components capable of generating a peritoneal dialysate from constituent parts.

The term "dialysate prescription" refers to the concentration of one or more solutes in peritoneal dialysate intended for use by a patient.

The term "downstream" refers to a position of a first component in a flow path relative to a second component wherein fluid, gas, or combination thereof, will pass by the second component prior to the first component during normal operation. The first component can be said to be "downstream" of the second component, while the second component is "upstream" of the first component.

An "enzyme-based sensor" is a component that measures a concentration of a first substance by catalytically converting the first substance to a second substance and measuring the amount of the second substance produced.

A "flow meter" is a device capable of measuring an amount or rate of fluid moving past or through a particular location.

The term "fluid" can be any substance without a fixed shape that yields easily to external pressure such as a gas or a liquid. Specifically, the fluid can be water containing any solutes at any concentration. The fluid can also be dialysate of any type including fresh, partially used, or spent.

The terms "fluid connection," "fluidly connectable," or "fluidly connected" refer to the ability to pass fluid or gas from one point to another point. The two points can be within or between any one or more of compartments, modules, systems, and components, all of any type.

A "fluid line" can refer to a tubing or conduit through which a fluid, gas, or fluid containing gas can pass. The fluid line can also contain air during different modes of operation such as cleaning or purging of a line.

The term "generating" or to "generate" refers to creating a substance or fluid from constituent parts.

The term "generating an alert" or to "generate an alert" can refer to generating or signaling to a user a state or condition of a system.

The terms to "generate peritoneal dialysate," "generating peritoneal dialysate" or "peritoneal dialysate generation" refers to creating a peritoneal dialysate solution from constituent parts.

The terms "heating" or to "heat" refer to raising the temperature of a substance, fluid, gas, or combinations of fluid and gas. The term can also refer to raising the temperature of a component such as container or a fluid line as described herein.

The term "infusate line" refers to a fluid line for carrying peritoneal osmotic agents and/or cation infusates into a peritoneal dialysate generation flowpath.

An "integrated cycler" is a component for movement of fluid into and out of the peritoneal cavity of a patient, wherein the integrated cycler forms a part of an overall system. For example, the integrated cycler can be contained in a housing with other components used for peritoneal dialysis and be in fluid and electrical connection with desired components.

An "ion concentrate" refers to one or more ionic compounds. The ion concentrate can have one or more ionic compounds in the ion concentrate. Further, the ion concentrate can have an ion concentration greater than an ion concentration to be used in dialysis.

An "ion concentrate source" refers to a source of one or more ionic compounds. The ion concentrate source can be in water or solid form. The ion concentrate source can further have one or more ionic compounds that are at a higher ion concentration greater than generally used in dialysis.

The term "measuring" or "to measure" can refer to determining any parameter or variable. The parameter or variable can relate to any state or value of a system, component, fluid, gas, or mixtures of one or more gases or fluids.

An "osmotic agent" is a substance dissolved in water capable of driving a net movement of water by osmosis across a semi-permeable membrane due to concentration differences of the osmotic agent on each side of the semi-permeable membrane.

The term "osmotic agent concentration" refers to an amount of an osmotic agent dissolved in a given volume of a fluid.

The term "osmotic agent flow rate" refers to a rate of fluid movement from an osmotic agent source.

An "osmotic agent source" refers to a source of osmotic agents in solid and/or solution form. The osmotic agent source can interface with at least one other module found in systems for dialysis. The osmotic agent source can contain at least one fluid pathway and include components such as conduits, valves, filters or fluid connection ports, any of which are fluidly connectable to each other or to a fluid flow path. The osmotic agent source can either be formed as a stand-alone enclosure or a compartment integrally formed with an apparatus for dialysis for containing an osmotic agent source. If the osmotic agent(s) is in solid form, a system as described in the present invention can deliver a fluid, such as a highly purified or sterile water, to dilute the solid osmotic agent. Optional mechanical agitation or other means such as stirring can be used to help dissolve the solid osmotic agent.

"Peritoneal dialysate" is a dialysis solution to be used in peritoneal dialysis having specified parameters for purity and sterility. Peritoneal dialysate is not the same as dialysate used in hemodialysis although peritoneal dialysate may be used in hemodialysis.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

A "pH sensor" is a component capable of measuring a concentration of hydrogen ions in a fluid.

The term "predetermined range" is a range of possible values for a parameter to be set as.

A "pulsed amperometric detection sensor" is a component that measures a concentration of a substance by applying an electrical potential to a sample, resulting in oxidation or reduction of the substance.

The term "pump" refers to any device that causes the movement of fluids or gases by applying suction or pressure.

The terms "pumping fluid" or to "pump fluid" refer to moving a fluid or gas through a flow path with a pump.

"Purified water" can be defined as water produced by distillation, deionization, reverse osmosis, or other suitable processes and that meets the definition of "purified water" in the United States Pharmacopeia, 23d Revision, January 1, 1995, and the FDA at 21 CFR Section § 165.110(a)(2)(iv). Other criteria for purified water can be determined by those of skill in the art, particularly as relating to purified water suitable for peritoneal dialysis.

A "refractive index sensor" is a component that measures the speed of light in a substance relative to the speed of light in a vacuum.

The term "secondary" as used in relation to a component is meant to distinguish two similar components and is not intended to describe the structure or function of the component being described as "secondary.".

The term "selecting" or to "select" refers to choosing a variable or parameter from a set of possible variables or parameter.

"Setting," "to set," and the like, can refer to an adjustment of any parameter, component, or algorithm to any particular value or position. The adjustment can include adjustment in any manner such as positioning a component, performing a physical act, or bringing any parameter, computer, algorithm, or computer into a particular state whether implemented by hand, a processor, a computer, or algorithm.

A "sterilization module" can be a component or set of components to sterilize a fluid, gas, or combination thereof by removing or destroying chemical or biological contaminants.

The term "upstream" refers to a position of a first component in a flow path relative to a second component wherein fluid, gas, or combinations thereof, will pass by the first component prior to the second component during normal operation. The first component can be said to be "upstream" of the second component, while the second component is "downstream" of the first component.

A "valve" can be a device capable of directing the flow of fluid or gas by opening, closing or obstructing one or more pathways to allow the fluid or gas to travel in a path. One or more valves configured to accomplish a desired flow can be configured into a "valve assembly."

The term "water purification module" refers to a component or components capable of removing biological or chemical contaminants from water.

The term "water source" refers to a source from which potable water can be obtained.

### Peritoneal Dialysis Preparation and Sensor System

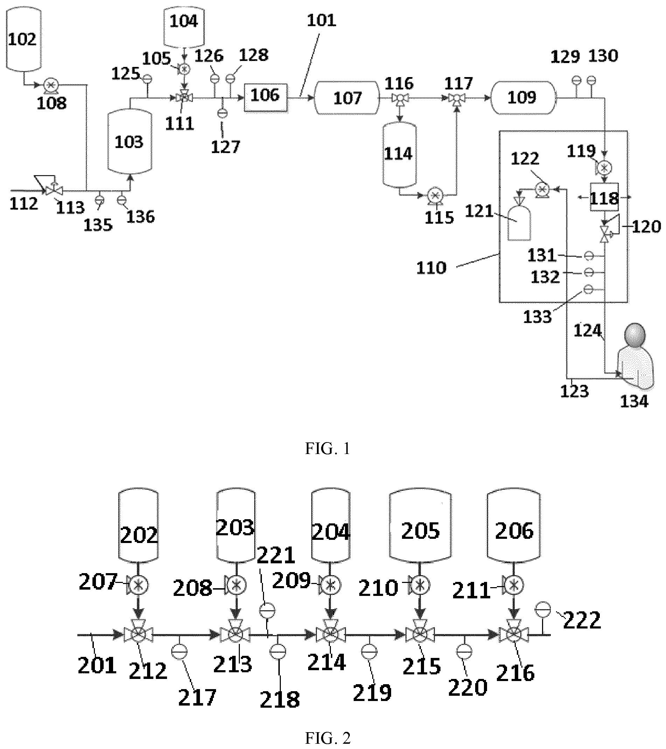

The invention relates to systems and methods for generating and using peritoneal dialysate in peritoneal dialysis. A system for generating peritoneal dialysate and delivering peritoneal dialysis therapy to a patient **134** can be configured as illustrated in FIG. 1. The system includes a peritoneal dialysate generation flow path **101.** Fluid from a water source, such as water tank **102,** can be pumped into the peritoneal dialysate generation flow path **101.** Additionally, or as an alternative to a water tank **102,** the system can use a direct connection **112** to a water source. System pump **108** can control the movement of fluid through the peritoneal dialysate generation flow path **101.** If a direct connection **112** to a water source is used, a pressure regulator **113** ensures the incoming water pressure is within a predetermined range. The system pumps the fluid from water source through a water purification module **103** to remove chemical contaminants in the fluid in preparation for creating dialysate.

The water source can be a source of potable water including a purified water source. Purified water can refer to any source of water treated to remove at least some biological or chemical contaminants. The water tank **102** can alternatively be a non-purified water source, such as tap water, wherein the water from the water tank **102** can be purified by the system as described. A non-purified water source can provide water that has undergone no additional purification, water that has undergone some level of purification, but does not meet the definition of "purified water" provided, such as bottled water or filtered water. The peritoneal dialysate generation flow path **101** can also have a direct connection **112** to a purified or non-purified water source, shown as direct connection **112.** The water source can be any source of water, whether from a tap, faucet, or a separate container or reservoir.

The water purification module **103** can be a sorbent cartridge. The sorbent cartridge can include aluminum oxide for removal of fluoride and heavy metals. The sorbent cartridge can have a first layer of aluminum oxide, a second layer of activated carbon and a third layer of an ion exchange resin. The sorbent cartridge can be sized depending on the needs of the user, with a larger sized sorbent cartridge allowing for more exchanges before the sorbent cartridge must be replaced. The sorbent cartridge can also include activated carbon. The activated carbon operates to adsorb non-ionic molecules, organic molecules, and chlorine from the water, along with some endotoxins or bacterial contaminants. In certain embodiments, the sorbent cartridge can include activated carbon, activated alumina, and potentially other components that work primarily by physical and chemical adsorption, combined with one or more ion exchange materials. The ion exchange materials can be any known material in the art, but preferably the ion exchange materials will release hydrogen and hydroxyl ions in exchange for other cations and anions in solution, resulting in water formation by the exchange process.

The sorbent cartridge can additionally include a microbial filter and/or a particulate filter. A microbial filter can further reduce the amount of bacterial contaminants present in the fluid from the water tank **102** or direct connection **112.** Optionally, an ultrafilter can be included to remove endotoxins from the fluid. A particulate filter can remove particulate matter from the fluid. The water tank **102** can be any size usable with the system, including between around 12 and around 25 L. A water tank **102** of 20 L can generally generate the necessary peritoneal dialysate for multiple cycles. In certain embodiments, the water purification module **103** can include an optional UV light source for further purification and sterilization of the water prior to adding osmotic agents or ion concentrates.

Alternatively, the water purification module **103** can be any component capable of removing contaminants from the water in the water source, including any one or more of a sorbent cartridge, reverse osmosis module, nanofilter, combination of cation and anion exchange materials, activated carbon, activated alumina, silica, or silica based columns.

After the fluid passes through the water purification module **103,** the fluid is pumped to a concentrate source **104,** where necessary components for carrying out peritoneal dialysis can be added from the concentrate source **104.** The concentrates in the concentrate source **104** are utilized to create a peritoneal dialysis fluid that matches a dialysate prescription. Concentrate pump **105** and concentrate valve **111** can control the movement of concentrates from the concentrate source **104** to the peritoneal dialysate generation flow path **101** in a controlled addition. Concentrate valve **111** can be replaced with a hose T. A hose T is a fluid connector in a T-shape, with a port at each end for fluid to enter or exit the hose T. The concentrates added from the concentrate source **104** to the peritoneal dialysate generation flow path **101** can include any component prescribed for use in peritoneal dialysate. Table 1 provides non-limiting exemplary ranges of commonly used components of peritoneal dialysate.

**Table 1**

| **Component** | **Concentration** |
|---|---|
| Sodium chloride | 132-134 mmol/L |
| Calcium chloride dehydrate | 1.25-1.75 mmol/L |
| Magnesium chloride hexahydrate | 0.25-0.75 mmol/L |
| Sodium Lactate | 35-40 mmol/L |
| Dextrose (D-glucose) monohydrate | 0.55-4.25 g/dL |
| pH | 5-6 |
| Osmolality | 346-485 (hypertonic) |

To reduce the glucose degradation products (GDP) formed, some peritoneal dialysate systems use a low GDP formulation. Exemplary peritoneal dialysate concentrations for low GDP formulations are provided in Table 2. Generally, the low GDP peritoneal dialysate is provided in two separate bags, with one bag containing calcium chloride, magnesium chloride and glucose maintained at low pH, and the second bag containing sodium chloride and the buffer components, including sodium lactate and sodium bicarbonate. The two bags are mixed prior to use to generate a peritoneal dialysate with a neutral pH. Alternatively, a two-chamber bag can be used to prevent mixing of fluids prior to use wherein the chambers, can for example, be separated by a wall of a divider of any type.

**Table 2**

| Low GDP peritoneal dialysate formulations | |
|---|---|
| **Component** | **Concentration** |
| Sodium | 132-134 mEq/L |
| Calcium | 2.5-3.5 mEq/L |
| Magnesium | 0.5-1.0 mEq/L |
| Lactate | 0-40 mEq/L |
| Bicarbonate | 0-34 mEq/L |
| pH | 6.3-7.4 |
| % glucose (g/dL) | 1.5-4.25 |

One of skill in the art will understand that other components can be used in place of the components listed in Tables 1-2. For example, dextrose as listed in Table 1 is commonly used as an osmotic agent. However, other osmotic agents can be used in addition to, or in place of, the dextrose, including glucose, icodextrin or amino acids, including dialysate with multiple osmotic agents. Although the sources of sodium, calcium, and magnesium listed in Table 1 are chloride salts, other sodium, magnesium, and calcium salts can be used, such as lactate or acetate salts. Peritoneal dialysate may also contain buffers for maintaining pH of the peritoneal dialysate, including bicarbonate buffer, acetate buffer, or lactate buffer. Although not generally used in peritoneal dialysis, potassium chloride can be used for hypokalemic patients who don't receive sufficient potassium through diet. The concentrate source **104** can contain one or more osmotic agents, as well as one or more ion concentrates, such as concentrated sodium chloride, sodium lactate, magnesium chloride, calcium chloride, and/or sodium bicarbonate. The concentrate source **104** can be a single source of concentrates, including both osmotic agents and ion concentrates, or can include multiple sources of concentrates, with separate sources for the osmotic agents and ion concentrates. The system can have a single concentrate that has all components mixed for a daytime or overnight treatment for use in a home by a single patient. Alternatively, the concentrate source **104** can include separate sources for any solutes to be used in the peritoneal dialysate each with a separate concentrate pump to add each solute. The ion concentrate source can be contained in an vessel or container of any type. The ion concentrate source can either be formed as a stand-alone enclosure or a compartment integrally formed with an apparatus for dialysis for containing an ion concentrate source.

Concentrate pump **105** pumps concentrated solutions from the concentrate source or sources **104** to the peritoneal dialysate generation flow path **101** in a controlled addition. Where more than one concentrate source is used, separate concentrate pumps can move each of the concentrates into the peritoneal dialysate generation flow path **101,** or a single concentrate pump can be used, with valves configured allow individual control over the movement of each of the concentrate solutions to the peritoneal dialysate generation flow path **101.**

After addition of solutes from the concentrate source **104,** the fluid in the peritoneal dialysate generation flow path **101** can contain all the necessary solutes for peritoneal dialysis. The peritoneal dialysate should reach a level of sterility for peritoneal dialysis. The level of sterility can be any level that meets an applicable regulatory requirement, such as a sterility assurance level of 10⁻⁶ required by the FDA, meaning that the chance a viable organism is present after sterilization is 1 in 1,000,000. The system can pump the fluid to a sterilization module for sterilization of the peritoneal dialysate. As shown in FIG. 1, the sterilization module can include one or more of a first ultrafilter **107,** a second ultrafilter **109,** and an optional UV light source **106.** The sterilization module can be any component or set of components capable of sterilizing the peritoneal dialysate. The sterilization module can be comprised of single or multiple ultrafilters. The number of ultrafilters can vary from one, two, three, four, and more depending on configuration and usage. A secondary component, such as a UV light source **106** or microbial filter (not shown), can be used in the sterilization module to provide additional sterilization of the peritoneal dialysate. The sterilization module can also include at least two ultrafilters, including second ultrafilter **109** for further sterilization of the fluid and redundancy of the system to protect against sterilization failure. The UV light source **106** can be positioned at any location in the peritoneal dialysate generation flow path **101,** including upstream of ultrafilter **107,** between ultrafilters **107** and **109** or downstream of ultrafilter **109.** The ultrafilters **107** and **109** used in the sterilization module can be replaced as necessary. In one non-limiting embodiment, the ultrafilters **107** and **109** can have a 3-6 month lifetime before replacement. However, no limitation on the lifespan of the ultrafilters is imposed by the system. The ultrafilters **107** and **109** can be any ultrafilter known in the art capable of sterilizing the peritoneal dialysate. A non-limiting example of an ultrafilter is the hollow fiber ForClean ultrafilter, available from Bellco, Mirandola (MO), Italy. In certain embodiments, the sterilization module **106** can use heat sterilization. The sterilization module can include a heater (not shown) to heat the prepared dialysate. Alternatively or additionally, the sterilization module can include a flash pasteurization module (not shown) to sterilize the dialysate through flash pasteurization. The sterilization module can include both heat-based sterilization components and filtration based sterilization components, with a processor, controller, or user adjusting the mode of sterilization based on the mode of use. For example, a heat based sterilization can be used when the peritoneal dialysate is generated for later use, while a filtration based sterilization can be used when the peritoneal dialysate is generated for immediate use.

The generated peritoneal dialysate can be pumped directly to an integrated cycler **110** for immediate infusion into a patient **134.** Alternatively, the dialysate can be pumped to an optional dialysate container **114** as a pre-prepared bolus of solution for storage until ready for use by a patient **134.** Valve **116** can control the movement of fluid to either the integrated cycler **110** or the dialysate container **114.** Stored dialysate in dialysate container **114** can be pumped as needed to the integrated cycler **110** by pump **115** through valve **117.** The dialysate container **114** can include one or more sterilized dialysate bags. The dialysate bags, once filled with peritoneal dialysate, can be stored until needed by the patient **134.** The dialysate container **114** can alternatively be a reusable sterilized container or bag. The reusable container or bag can be cleaned and sterilized daily, or at set time periods. Alternatively, the dialysate container **114** can be any type of storage container, such as a stainless-steel container. The dialysate container **114** can store enough peritoneal dialysate for a single infusion cycle of peritoneal dialysate into the patient **134,** or enough peritoneal dialysate for multiple infusions into a patient **134.** Additional or alternative storage containers can be included at other locations in the peritoneal dialysate generation flow path **101.** A storage container can be included upstream of the sterilization module, and downstream of the water purification module **103.** Before the fluid is utilized in the cycler stage, the fluid can be pumped through the sterilization module to ensure sterility of stored fluid. Further, concentrates can be added to fluid before storing the fluid, or after storage of the fluid but prior to sterilization in the sterilization module.

The storage containers can be either upstream or downstream of the concentrate source **104.** The addition of concentrates to the fluid can happen either before storage of the fluid, or after storage of the fluid just before sterilization in the sterilization module.

By generating and immediately using the peritoneal dialysate, the dialysate storage time can be reduced, reducing the possibility of bacterial growth. A user interface can be included on the peritoneal dialysis generation machine in communication with the control system, allowing a patient **134** to direct the generation of peritoneal dialysate at a selected time as needed. Additionally, or alternatively, the peritoneal dialysate machine can include a timer, and the timer can cause the peritoneal dialysate machine to generate peritoneal dialysate at predetermined times according to the patient's **134** peritoneal dialysis schedule. Alternatively, the peritoneal dialysate generation machine can be equipped with wireless communication, such as Wi-Fi, Bluetooth, Ethernet, or any other wireless communication system known in the art. The user can direct the peritoneal dialysis machine to generate peritoneal dialysate at a specified time from any location. By using a timer, user interface, or wireless communication to control the generation of peritoneal dialysate on demand, the peritoneal dialysate storage time can be reduced, lowering the chances of generating significant amounts of degradation products or allowing bacterial growth.

The peritoneal dialysate can be generated and used in real time, with direct infusion of the peritoneal dialysate into the patient **134** through the integrated cycler **110.** For real time generation and use of the peritoneal dialysate, the flow rate of fluid through the peritoneal dialysate generation flow path **101** can be between 50 and 300 ml/min. With the online generation of fluid described, a flow rate of 300 ml/min can support an exchange time of between 10 and 15 minutes for a full cycle of draining and filling the peritoneal cavity of a patient **134.** If a dialysate container **114** is used to store generated peritoneal dialysate, the flow rate of fluid through the peritoneal dialysate generation flow path **101** can be any flow rate capable of producing the necessary peritoneal dialysate. In certain embodiments, the flow rate can be at least around 15 mL/min, which will produce 20 L of peritoneal dialysate in 24 hrs. The integrated cycler **110** can then infuse the generated peritoneal dialysate into the peritoneal cavity of a patient **134.** The integrated cycler **110** and the rest of the system can communicate for the purposes of generation and use of the peritoneal dialysate by any method known in the art, including Bluetooth, Wi-Fi, Ethernet, or direct hardware connections to meet patient or clinic needs. Additional valves and regulators (not shown in FIG. 1) can be included to aid in connection and operation of the peritoneal dialysate generation flow path **101** and integrated cycler **110.** The integrated cycler **110** and the peritoneal dialysate generation flow path **101** can communicate directly, or can each communicate with a control system for control over the generation and use of the peritoneal dialysate.

In certain embodiments, the dialysate container **114** can store enough peritoneal dialysate for multiple infusions into the patient **134,** including enough peritoneal dialysate for one day or more of treatment. A timer can be included in the control system and can cause the machine to generate fresh peritoneal dialysate each day or at set times.

The integrated cycler **110** can include a metering pump **119** for metering peritoneal dialysate into the peritoneal cavity of the patient **134.** An in-line heater **118** heats the peritoneal dialysate to a desired temperature prior to infusion into the patient **134.** A pressure regulator **120** ensures the peritoneal dialysate pressure is within a predetermined range safe and comfortable for infusion into the patient **134.** The metering pump **119** can use any safe pressure for infusing fluid into the patient **134.** Generally, the pump pressures are on average set at ±10.3 kPa or 77.6 mmHg. If there is no fluid flow, the maximum pressure can increase to ±15.2 kPa or 113.8 mmHg for a short period, such as less than 10 seconds. The peritoneal dialysate is infused into the peritoneal cavity of the patient **134** through infusion line **124.** An additional microbial filter (not shown) may be used to sterilize the peritoneal dialysis fluid immediately before the peritoneal dialysate enters the patient **134.** After a dwell period, the peritoneal dialysate is drained from the patient **134** through drain line **123.** Pump **122** provides a driving force for removing the peritoneal dialysate from the patient **134.** Treatment, other than the first full cycle for a patient in APD, generally begins with drainage of the peritoneal cavity of the patient **134,** prior to infusing the fresh peritoneal dialysate into the patient **134.** An optional waste reservoir **121** can be included to store the used peritoneal dialysate for disposal. Alternatively, the drain line **123** can be directly connected to a drain for direct disposal. A standard waste reservoir **121** is 15 L, however, the waste reservoir **121** can be any size, including between 12 and 20 L. For patients requiring a higher drainage, a drain manifold can be included for connecting multiple waste reservoirs. There is no set rate for draining of peritoneal dialysate from the peritoneal cavity of the patient **134,** and any flow rate can be used with the integrated cycler **110.**

Various sensors positioned in the peritoneal dialysate generation and infusion system ensure that the generated fluid is within predetermined parameters. Flow meter **135** ensures the incoming water is at a correct flow rate, while pressure sensor **136** ensures the incoming water is at an appropriate pressure. Conductivity sensor **125** is used to ensure that the water exiting water purification module **103** has been purified to a level safe for use in peritoneal dialysis. Conductivity sensor **126** ensures the conductivity of the dialysate after the addition of concentrates from concentrate source **104** is within a predetermined range. Refractive index sensor **127** ensures that the concentration of the osmotic agents is within a predetermined range. pH sensor **128** ensures the pH of the peritoneal dialysate is within a predetermined range. After passing through the sterilization module including second ultrafilter **109,** pH sensor **129** and conductivity sensor **130** are used to ensure that no changes in the pH or conductivity have occurred during purification or storage of the dialysate in dialysate container **114.** The integrated cycler **110** has flow meter **131,** pressure sensor **132** and temperature sensor **133** to ensure that the dialysate being infused into the patient **134** is within a proper flow rate, pressure, and temperature range. The flow meter **131** can also calculate the volume of solution infused into the patient **134.** The pressure sensor **132** can monitor the pressure in the peritoneal cavity.

Overfill, or excessive solution in the peritoneal cavity beyond the target volume may present complications in therapy. Overfill can be caused by many factors, including failing to fully drain the peritoneal cavity prior to infusion of fresh peritoneal dialysate. In any embodiment, the integrated cycler **110** can start therapy with a drain step to ensure that no peritoneal dialysate remains in the peritoneal cavity. Monitoring both pressure and volume of peritoneal dialysate introduced to the patient **134** can avoid overfill. If the pressure rises to a certain point, the system can be programmed to end filling or send an alert to a user to complete filling of the peritoneal cavity at a desired level. The volume of peritoneal dialysate extracted from and introduced to the patient **134** can also be monitored with flow meters to ensure proper volumes of exchanges. Draining the peritoneal cavity can be performed in a similar manner by monitoring the pressure and volume of the drained peritoneal dialysate.

As illustrated in FIG. 1, the necessary solutes can be added to the peritoneal dialysate generation flow path **101** from a single concentrate source **104.** The solutes can be present in concentrated from within the concentrate source **104** in a fixed ratio for peritoneal dialysis, as shown in Table 1. Using a single concentrate source **104** for all solutes results in peritoneal dialysate having a fixed ratio of each of the solutes.

Table 3 provides exemplary non-limiting ranges of solutes that can be added from a single concentrate source **104** to the peritoneal dialysate generation flow path **101,** including the starting concentration of the solutes in the concentrate source, as well as exemplary final volumes of the solutes in the dialysate and the exemplary flow rates of both the solutes and the water in the peritoneal dialysate generation flow path **101** that will achieve those concentrations. The solutes shown in Table 3 are traditional peritoneal dialysate solutes. Table 4 shows exemplary ranges of solutes that can be used as a low GDP formulation. Table 5 shows exemplary ranges of solutes that can be used with icodextrin as the osmotic agent. Icodextrin is sometimes used as an osmotic agent for a long dwell period. If dextrose or glucose is used in a long dwell period, reabsorption of the ultrafiltrate can occur, reducing the net volume of fluid removed. Icodextrin results in a long sustained ultrafiltration, and can provide improved ultrafiltration efficiency over a long dwell period. One of skill in the art will understand that the concentrations of any of the solutes shown in Tables 3-5 can be altered by altering the flow rates of the system pump **108** or concentrate pump **105.** However, the ratio of the solutes included is fixed if using a single concentrate source **104.** If the ratio of the solutes needs to be altered for any reason, a new concentrate solution may be needed.

**Table 3**

| Exemplary solutes for addition from a single concentrate source | | | |
|---|---|---|---|
| **Component** | **Concentration (g/l)** | **Solution volume (ml/L)** | **Flow rate (ml/min)** |
| Glucose | 100 - 850 | 50-400 | 1 - 18 |
| Sodium Chloride | 13 - 108 | 50-400 | 1 - 18 |
| Sodium Lactate | 11 - 90 | 50-400 | 1 - 18 |
| MgCl₂.6H₂O | 0.13 - 1.02 | 50-400 | 1 - 18 |
| CaCl₂.2H₂O | 0.6 - 5.1 | 50-400 | 1 - 18 |
| Water | | 600-950 | 50-1000 |

**Table 4**

| Exemplary solute ranges in a low GDP solution | | | |
|---|---|---|---|
| **Component** | **Concentration (g/l)** | **Solution volume (ml/L)** | **Flow rate (ml/min)** |
| Glucose | 100 - 900 | 50-400 | 1 - 18 |
| Sodium Chloride | 13 - 108 | 50-400 | 1 - 18 |
| Sodium Lactate | 11 - 90 | 50-400 | 1 - 18 |
| MgCl₂.6H₂O | 0.13 - 1.02 | 50-400 | 1 - 18 |
| CaCl₂.2H₂O | 0.6 - 5.1 | 50-400 | 1 - 18 |
| Water | | 600-950 | 50-1000 |

**Table 5**

| Exemplary solute ranges in icodextrin solution | | | |
|---|---|---|---|
| **Component** | **Concentration (g/l)** | **Solution volume (ml/L)** | **Flow rate (ml/min)** |
| Icodextrin | 100 - 850 | 100-400 | 2-37 |
| Sodium Chloride | 13 - 108 | 100-400 | 1 - 18 |
| Sodium Lactate | 11 - 90 | 100-400 | 2-37 |
| MgCl₂.6H₂O | 0.13 - 1.02 | 100-400 | 2-37 |
| CaCl₂.2H₂O | 0.6 - 5.1 | 100-400 | 2-37 |
| Water | | 600-900 | 50-1000 |

Although using a single concentrate source **104** in the system requires a fixed ratio of solutes in the generated peritoneal dialysate, a single concentrate source **104** provides certain advantages. Storage requirements are decreased, as only a single concentrate solution needs to be stored for a given dialysate prescription. There is also a lower risk of patient error in adding solutes to the dialysate in the proper amounts. A single concentrate source **104** also requires less supplies, less pumps, and less hardware. Further, because fewer containers are needed, the containers are easier to manage, clean, and disinfect. A higher concentration of solutes in the concentrate source **104** will allow minimization of the container size and maximization of the source water used in PD solution preparation, lowering costs. The limiting factor is mutual solubility of the components, which is generally limited by glucose or icodextrin solubility. The flow rate for the source water can be optimized to adjust the time required to prepare the solution. In the case of on-demand dialysate preparation, a high flow rate is desired to minimize the time needed to prepare the solution. The flow rate limit will be controlled by the metering accuracy of the concentrate pump **105** at the rate required to match the water feed. With a single concentrate source **104,** about 150 ml/exchange can be needed, which corresponds to about 600 ml/day or 4.2 L/week. The concentrate source **104** can be sized depending on the needs of the user, with a larger concentrate source requiring less frequent refilling.

The system can also include an additional waste reservoir (not shown in FIG. 1) to collect any waste fluid generated by the water purification module **103** or other components. Alternatively, waste reservoir **121** can also be used to collect any waste fluid generated by the water purification module **103** or other components. The waste reservoir **121** collects effluent generated during disinfection and/or effluent generated by the purification modules, such as a reverse osmosis system.

The peritoneal dialysate generation flow path **101** and integrated cycler **110** can be disinfected with a disinfection solution through on-board disinfection if the components of the peritoneal dialysate generation flow path **101** and integrated cycler **110** are to be reused. Disinfection may not be required with a fully disposable peritoneal dialysate generation flow path **101.** The peritoneal dialysate generation flow path **101** and integrated cycler **110** can be configured to form a loop by connecting the portion of the peritoneal dialysate generation flow path **101** that connects to water tank **102** or the direct connection **112** to a water source to the infusion line **124.** The disinfection solution can be introduced into the peritoneal dialysate generation flow path **101** and recirculated through the fluid lines by system pumps **108** and **119.** Alternatively, the peritoneal dialysate generation flow path **101** and integrated cycler **110** can be disinfected separately after disconnection of the integrated cycler **110** from the peritoneal dialysate generation flow path **101.** The disinfection solution can be a citric acid solution, a peracetic acid solution, a bleach solution, or any other disinfection solution known in the art. Disinfectant can be circulated through the flow loop and heated. The disinfectant can be heated to any temperature capable of disinfecting the system, including temperatures of at least 80 °C or greater. The disinfectant can be introduced to the flow loop and recirculated at elevated temperatures to ensure complete disinfection.

Solutes can be added to the peritoneal dialysate generation flow path **201** from two or more separate concentrate sources, as shown in FIG. 2. The peritoneal dialysate generation flow path **201** can be fluidly connected to a water source and a water purification module upstream of the concentrate sources **202-206,** and a sterilization module, an integrated cycler, and optionally a dialysate container downstream of the concentrate sources **202-206,** as illustrated in FIG. 1. For clarity, these components have been omitted from FIG. 2.

As illustrated in FIG. 2, the concentrate sources **202-206** can include one or more ion concentrate sources, such as sodium chloride source **202** containing sodium chloride to be added in a controlled addition to the peritoneal dialysate generation flow path **201** by concentrate pump **207** through valve **212,** sodium lactate source **203** containing sodium lactate to be added in a controlled addition to the peritoneal dialysate generation flow path **201** by concentrate pump **208** through valve **213,** magnesium chloride source **204** containing magnesium chloride to be added in a controlled addition to the peritoneal dialysate generation flow path **201** by concentrate pump **209** through valve **214,** and calcium chloride source **205** containing calcium chloride to be added in a controlled addition to the peritoneal dialysate generation flow path **201** by concentrate pump **210** through valve **215.** One of skill in the art will understand that other ions can be used in formulation of peritoneal dialysate, and each can be contained in a separate ion concentrate source or combined into one or more combined ion concentrate sources. The concentrate source also includes one or more osmotic agent sources, such as dextrose source **206** containing dextrose to be added to the peritoneal dialysate generation flow path **201** by concentrate pump **211** through valve **216.** Any of the concentrate pumps can include flow meters to control the addition of the solutes. A glucose source and/or an icodextrin source can be used in addition to, or in place of, dextrose source **206.** Multiple osmotic agents can be added to the peritoneal dialysate generation flow path **201** from one or more osmotic agent sources. One of skill in the art will understand other solutes can be used alternatively to, or in addition to, the solutes illustrated in FIG. 2. A control system in electronic communication with each of the concentrate pumps can control the movement of fluid from the concentrate sources to the peritoneal dialysate generation flow path **201.** The amount of each of the concentrates moved into the peritoneal dialysate generation flow path **201** can be controlled to result in peritoneal dialysate having a prescribed solute concentration, as determined by a doctor or health care provider. The valves **212-216** can optionally be replaced with hose T junctions with additional components for preventing backflow into the concentrate source line if that particular line is not being used. Optional sensors **217, 218, 219,** and **220** ensure the solute concentration in the dialysate is at the correct level after each addition. The sensors **217-220** can be any type of sensor appropriate to confirm delivery of the concentrate, such as conductivity sensors. Optional pH sensor **221** can be used to ensure that the pH is a proper level after addition of sodium lactate or other buffer. Optional refractive index sensor **222** ensures the dextrose concentration in the dialysate is at the prescribed level. An additional sensor can be included upstream of sodium chloride source **202** for sensing the conductivity of the water prior to addition of concentrates. One of skill in the art will understand that additional sensor arrangements can be used in the described system. Any number of sensors can be included to monitor the peritoneal dialysate concentration, including 1, 2, 3, 4, 5, 6, 7, or more sensors. The concentrate sources can contain the solutes in either solid, powdered, or solution form. A solid or powdered source of solutes can be dissolved by the system by drawing fluid from the peritoneal dialysate generation flow path **201** into the concentrate source to generate a solution with a known concentration, such as a saturated solution of the solutes. During the process of dissolution of the solutes, agitating the concentrates by mechanical means, vibration, heating the concentrates, or other forms of assistance may be used to dissolve the solid or powder solutes. The resulting solution is added to the peritoneal dialysate generation flow path as explained. Although shown as a refractive index sensor **222** in FIG. 2, one of skill in the art will understand that alternative methods of measuring the osmotic agent concentration can be used, including enzyme based sensors or pulsed amperometric detection. Enzyme-based sensors can detect the concentration of the osmotic agent in the dialysate. Enzyme based sensors use an enzyme capable of oxidizing the osmotic agent, such as glucose or dextrose. The enzyme is immobilized on an electrode and covered in a membrane through which the osmotic agent can pass. The electrode is used to electrochemically measure the change in either the oxidant, such as oxygen, or the product of glucose oxidation, such as hydrogen peroxide. Alternatively, electron transfer between the electrode and the enzyme can be detected with mediators, such as ferrocene to facilitate electron transfer. The osmotic agents can alternatively be detected by a pulsed amperometric detection sensor (PAD). PAD can detect glucose by applying a positive potential to a sample, resulting in oxidation of the glucose. The oxidation products are adsorbed onto the electrode and then desorbed by applying a more positive potential. Applying the more positive potential results in formation of an oxide layer on the electrode leading to passivation of the electrode surface. The catalytic activity of the electrode is then restored by application of a more negative potential, resulting in dissolution of the oxide layer.

Although illustrated as a single concentrate source in FIG. 1, and five separate concentrate sources in FIG. 2, one of skill in the art will understand that any number of concentrate sources can generate the peritoneal dialysate, including 1, 2, 3, 4, 5, 6, 7, or more concentrate sources. Any two or more of the separate concentrate sources illustrated in FIG. 2 can be combined into a single solute source, such as by combining all or some of the ion concentrate sources into a single ion concentrate source where the mixed contents do not cause precipitation of the mixed concentrates. Although each concentrate source is illustrated in FIG. 2 with a separate concentrate pump and fluid line, one of skill in the art will understand that more than one concentrate source can use a single pump and fluid line, with valves arranged thereon for controlled addition to the peritoneal dialysate generation flow path **201.**

The concentrate sources **202-206** can be single use concentrate sources or disposable concentrate sources. The disposable concentrate sources are used in a single peritoneal dialysate generation process and then disposed. Multiple use concentrate sources are used repeatedly, and refilled as necessary with the solute.

Table 6 provides exemplary, non-limiting, ranges of solutes that can be added to the peritoneal dialysate using a separate osmotic agent source, glucose in Table 6, and a separate ion concentrate source containing sodium chloride, sodium lactate, magnesium chloride, calcium chloride and sodium bicarbonate. Because the glucose is added separately from the ion concentrates, the ratio of glucose to the other solutes can be varied depending on the needs of the patient.

**Table 6**

| Exemplary ranges of solutes in a two-concentrate source system | | | |
|---|---|---|---|
| **Component** | **Concentration (g/l)** | **Solution volume (ml/L)** | **Dialysate composition** |
| **Part A** | | | |
| Glucose | 850 | 6 - 53 | 0.55-4.5 g/dL |
| **Part B** | | | |
| NaCl | 269 | 20 | 92 mmol/L |
| Sodium Lactate | 84 | 20 | 15 mmol/L |
| MgCl₂.6H₂O | 5 | 20 | 0.5 mmol/L |
| CaCl₂.2H₂O | 18 | 20 | 2.5 mmol/L |
| NaHCO₃ | 105 | 20 | 25 mmol/L |
| Water | | 927-979 | 56.10 |

By using multiple concentrate sources, greater individualization and therapy customization can be achieved for each patient. With a single concentrate source, all solutes in the generated peritoneal dialysate must be present in a fixed ratio. By using more than one concentrate source, the ratio of solutes used in the peritoneal dialysate can be altered as the concentration of each of the osmotic agent and ion solutes can be individually controlled. For example, as illustrated by Table 6, with a single ion concentrate source and a single osmotic agent source, peritoneal dialysate with greater or less osmotic agent per concentration of ions can be generated, providing the ability to adjust the tonicity of the peritoneal dialysate solution independently of the electrolyte composition to meet the UF needs of any patient with a single set of solutions and allowing greater control over ultrafiltration. The ultrafiltration rate that results from using the peritoneal dialysate solutions can be altered by altering the concentration of the osmotic agent independently of the ionic solutes, or by changing the osmotic agent used. Because the system is not limited to discrete glucose or other osmotic agent concentrations like known commercial solutions; the system can customize the peritoneal dialysate solutions to meet the ultrafiltration needs of patient as determined by a healthcare provider. As illustrated in Table 6, the glucose level in the peritoneal dialysate solution can be varied from 0.55 g/dL to 4.5 g/dL, while maintaining the electrolytes and buffer components constant, allowing the system to cover the range of glucose formulations currently offered commercially using a single Part A and Part B composition.

In certain embodiments, two osmotic agent sources can be used, such as a dextrose source and an icodextrin source. With two osmotic agent sources, one could use dextrose during the daytime exchanges for CAPD and icodextrin during the night dwell to take advantage of the higher UF removal from icodextrin. Conversely, dextrose could be used during the night dwell and icodextrin for the extended daytime dwell in APD systems.

By using separate concentrate sources for each solute, complete individualization of the concentrations and ratios of solutes in the peritoneal dialysate can be achieved. Table 7 provides exemplary ranges of solutes that can be used in peritoneal dialysate as made by a system with each solute in a separate concentrate source. An advantage of using separate concentrate sources for each solute is that virtually any peritoneal dialysate solution composition can be prepared from a single set of component formulations. A system with separate concentrate sources for each solute is useful for patients whose prescriptions change periodically due to diet or other factors. Such patients would need to store multiple formulations if using only one or two concentrate sources, and the risk of errors would be increased.

**Table 7**

| Exemplary dialysate composition from a multi-source system | | | |
|---|---|---|---|
| **Component** | **Concentration (g/l)** | **Solution volume (ml/L)** | **Dialysate composition** |
| Part A: Glucose | 850 | 6 - 53 | 0.55-4.5 g/dL |
| Part B: NaCl | 320 | 15-18 | 132-134 mmol/L |
| Part C: Na Lactate | 1000 | 2-4 | 15-40 mmol/L |
| Part D: MgCl2.6H2O | 500 | 0.2-0.4 | 0.5 -1.0 mmol/L |
| Part E: CaCl2.2H2O | 700 | 0.5-1.0 | 2.5-3.5 mmol/L |
| Part F: NaHCO3 | 85 | 0-34 | 0-34 mmol/L |
| Part G: Icodextrin | 1000 | 0-75 | 0-7.5 g/dL |
| Water | | 820-971 | |

The one or more concentrate sources can be detachable from the rest of the system for sterilization. The concentrate sources can also be sterilized each time the concentrate sources are filled with new concentrate solutions. Further, the concentrate sources can be sterilized after a set number of uses, or after a set period of time. Moreover, the concentrate sources and the rest of the peritoneal dialysate generation system can be sterilized without any of the components by passing a disinfection solution, such as a citric acid, peracetic acid, or bleach solution, through all of the lines and containers of the system.

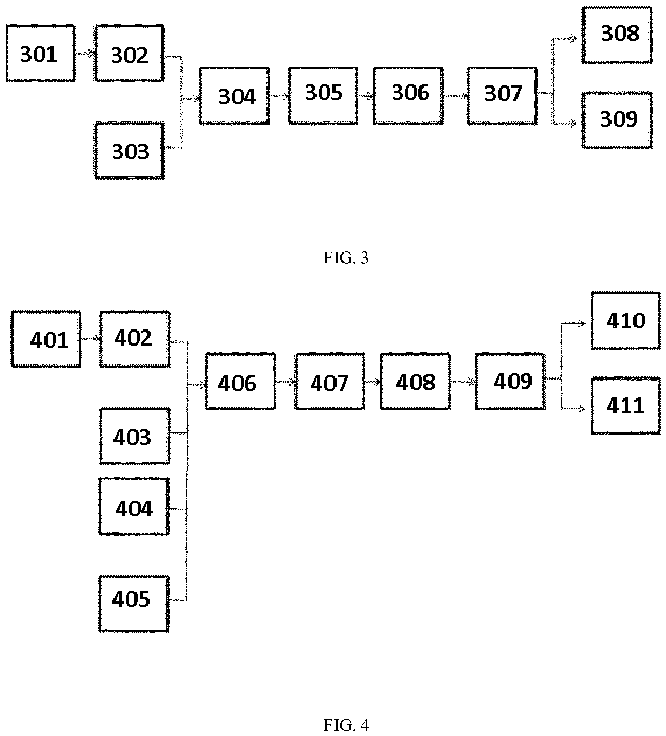

FIG. 3 illustrates an overview of generating peritoneal dialysate in accordance with any embodiment of the invention. Water from a water source **301** can be purified by a water purification module **302,** as explained. Concentrates from a single concentrate source **303,** which can contain both ion concentrates and one or more osmotic agents, can be added to the purified water to generate a non-sterile peritoneal dialysate solution **304.** The non-sterile peritoneal dialysate solution **304** is sterilized by a sterilization module **305,** which may include an ultrafilter (not shown). As explained, the peritoneal dialysate can be further purified by additional components in the sterilization module **306,** such as by ultrafiltration with a second ultrafilter, by a microbial filter, or by an optional UV light source, to generate a sterilized peritoneal dialysate **307.** The sterilized peritoneal dialysate **307** can be stored or used by any method described herein, including by immediately infusing the peritoneal dialysate into a patient **308,** or dispensing the peritoneal dialysate into a dialysate container for later use in peritoneal dialysis **309,** as illustrated in FIG. 1.

FIG. 4 illustrates an overview of generating peritoneal dialysate with multiple concentrate sources. Water from a water source **401** can be purified by a water purification module **402,** as explained. Concentrates from an ion concentrate source **403,** which can contain sodium, magnesium, calcium, and bicarbonate, as well as any other ions to be used in peritoneal dialysis, can be added to the purified fluid. An osmotic agent, such as dextrose, can be added from a first osmotic agent concentrate source **404.** A second osmotic agent, such as icodextrin, can be added from a second osmotic agent concentrate source **405.** As illustrated in FIG. 2, any number of concentrate sources can be used for further individualization of the peritoneal dialysate, including separate sources for each of the ions used. After addition of the ion and osmotic agent concentrates, the fluid contains all necessary components for use in peritoneal dialysis as non-sterilized peritoneal dialysate **406.** The non-sterile peritoneal dialysate **406** can be sterilized by a sterilization module **407,** which can include an ultrafilter or other sterilization components. The peritoneal dialysate can be further sterilized by the sterilization module **408,** either by ultrafiltration with a second ultrafilter, a microbial filter, or further sterilized with an optional UV light source, to generate a sterilized peritoneal dialysate **409.** The sterilized peritoneal dialysate **409** can be stored or used by any method described herein, including by immediately infusing the peritoneal dialysate into a patient **410,** or dispensing the peritoneal dialysate into a dialysate container for later use in peritoneal dialysis **411,** as illustrated in FIG. 1.

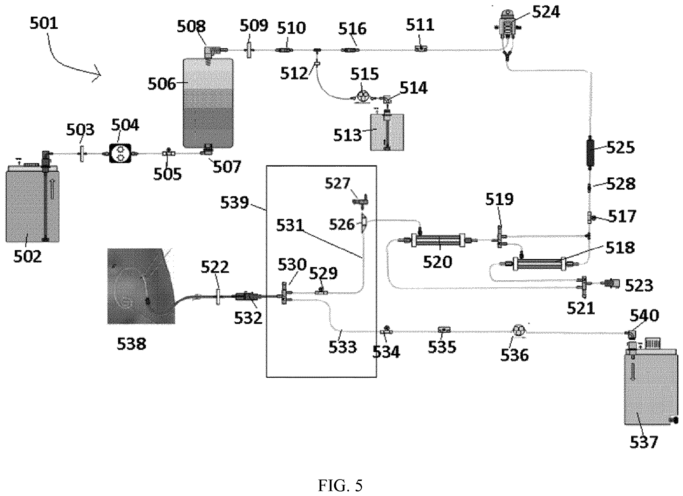

FIG. 5 illustrates an alternative peritoneal dialysate generation flow path **501** with an integrated cycler **539.** Water from a water source **502** can be pumped through filter **503** by system pump **504.** The filter **503** can remove any particulate matter from the water prior to entering the peritoneal dialysate generation flow path **501.** The water is then pumped through a water purification module, illustrated as a sorbent cartridge **506** in FIG. 5. As described, the water purification module can alternatively or additionally include activated carbon, a reverse osmosis module, a carbon filter, an ion exchange resin, and/or a nanofilter. The water enters the sorbent cartridge **506** through sorbent cartridge inlet **507** and exits through sorbent cartridge outlet **508.** Pressure sensor **505** measures the pressure across sorbent cartridge **506.** Filter **509** removes any particulate matter in the fluid after exiting sorbent cartridge **506.** A conductivity sensor **510** determines the conductivity of the fluid exiting sorbent cartridge **506** to ensure the water has been purified. To generate the peritoneal dialysate, concentrates are added from concentrate source **513** through concentrate connector **514** by concentrate pump **515.** Although shown as a single concentrate source **513** in FIG. 5, concentrates can be added from any number of separate concentrate sources. Concentrate filter **512** removes any particulate matter from the concentrate before entering the peritoneal dialysate generation flow path **501.** A conductivity sensor **516** determines the conductivity of the generated peritoneal dialysate after addition of the concentrates to ensure the peritoneal dialysate has the correct solute concentrations. Flow meter **511** determines the flow rate of the fluid after addition of the concentrates. pH sensor **524** determines the pH of the peritoneal dialysate to ensure the peritoneal dialysate has a proper pH. The peritoneal dialysate can be heated to a desired temperature by heater **525.** Temperature sensor **528** ensures the peritoneal dialysate is heated to an appropriate temperature before infusion into the patient **538.** The heater **525** can be placed at any location in the flow path prior to delivery to the patient **538.** In any embodiment, the heater **525** can be located after the exit of the sterilization module, particularly if fluid is stored prior to passing through the sterilization module. The desired temperature of the peritoneal dialysate can be between around 20°C to around 41°C. As used herein, around 20°C can include between 19.0°C and 21.0 °C, and around 41°C can include between 39.0°C and 41.0°C, or similar as understood by those of skill in the art. In certain embodiments, the desired temperature can be between around 25°C to around 40°C, around 36.5°C to around 37.25°C, around 25°C to around 35°C, or around 30°C to around 40°C. In a preferred embodiment, the desired temperature can be 37 ± 2 °C.

As described, the peritoneal dialysate is sterilized by pumping the peritoneal dialysate through a sterilization module, which can include first ultrafilter **518,** and optionally a second ultrafilter **520** and/or an optional UV light source (not shown). Pressure sensor **517** measures the fluid pressure prior to the fluid entering the sterilization module, shown as ultrafilters **518** and **520,** and is used in the control circuit to control the pressure. The fluid passes through first ultrafilter **518,** through valve **519,** and then through second ultrafilter **520.** Connector **523,** three way valve **521,** and valve **519** allow backflushing and disinfection of the ultrafilters **518** and **520.** The fluid is then pumped into the integrated cycler **539** for use in peritoneal dialysis. As described, the system can include a dialysate container (not shown) for storage of the generated peritoneal dialysate until used by the patient **538** at any location, including upstream or downstream of the sterilization module.

The integrated cycler **539** includes an infusion line **531** and a drain line **533.** Bubble trap **526** traps air bubbles present in the heated dialysate. The air is vented from the system through bubble trap valve **527.** Pressure sensor **529** ensures the pressure of the fluid is within a predetermined range. In certain embodiments, the predetermined range can be a pressure of between -200 mmHg to 500 mmHg, from -50 mmHg to 100 mmHg, from 0 mmHg to 100 mmHg, from -50 mmHg to 200 mm Hg, from 200 mmHg to 500 mmHg, or from 100 mmHg to 400 mmHg. The infusion line **531** is connected to a three-way valve **530,** which controls fluid movement between the infusion line **531,** the patient **538,** and the drain line **533.** The three way valve **530** is connected through connector **532** to a catheter inserted into the peritoneal cavity of the patient **538.** A filter **522** can be included between the three-way valve **530** and the catheter for additional cleaning of the peritoneal dialysate prior to entering a patient **538.** In any embodiment, the filter **522** can be a disposable filter. The peritoneal dialysate is infused into the patient **538** and held for a dwell period. After the dwell period, the fluid is pumped out of the peritoneal cavity of the patient **538** by drain pump **536.** The three-way valve **530** is switched to direct fluid into the drain line **533.** Pressure sensor **534** measures the pressure of fluid in the drain line **531** to ensure proper drainage. Flow meter **535** measures the flow rate and volume of fluid removed from the patient **538.** The drain line **531** is connected to a drain or waste reservoir **537** through connector **540** for collection and disposal of the used peritoneal dialysate.

For automated disinfection of the system, connector **540** can be connected to connector **523** to form a flow loop. Disinfectant can be circulated through the flow loop and heated. The disinfectant can be heated to any temperature capable of disinfecting the system, including temperatures of at least around equal to 80 °C or greater (≥80) when using citric acid as a disinfectant. Peracetic acid or bleach can be used to disinfect the system at room temperature. The disinfectant can be introduced to the flow loop and recirculated at elevated temperatures to ensure complete disinfection. The disinfectant used can be any suitable disinfectant known in the art, including peracetic acid, citric acid, or bleach. The connectors and components of the system can be gamma and autoclave compatible to resist the high temperatures used during disinfection. The system can be primed by introducing a priming fluid to the peritoneal dialysate generation flow path **501** and integrated cycler **539.**

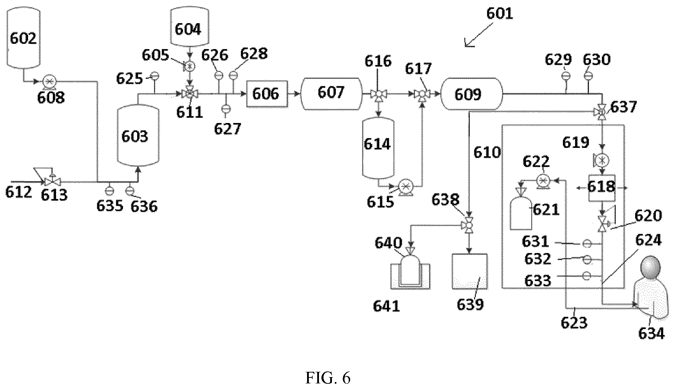

FIG. 6 illustrates an alternative embodiment of the system. Fluid from a water source, such as water tank **602,** can be pumped into the peritoneal dialysate generation flow path **601.** Additionally, or as an alternative to a water tank **602,** the system can use a direct connection to a water source **612.** System pump **608** can control the movement of fluid through the peritoneal dialysate generation flow path **601.** If a direct connection to a water source **612** is used, a pressure regulator **613** can ensure that an incoming water pressure is within a predetermined range. The system pumps the fluid from water source **602** or **612** through a water purification module **603** to remove chemical contaminants in the fluid in preparation for creating dialysate.

After the fluid passes through the water purification module **603,** the fluid is pumped to a concentrate source **604,** where necessary components for carrying out peritoneal dialysis can be added from the concentrate source **604.** The concentrates in the concentrate source **604** are utilized to create a peritoneal dialysis fluid that matches a dialysate prescription. Concentrate pump **605** and concentrate valve **611** can control the movement of concentrates from the concentrate source **604** to the peritoneal dialysate generation flow path **601** in a controlled addition. Alternatively, concentrate valve **611** can be a hose T or backflow restricting hose T. The concentrates added from the concentrate source **604** to the peritoneal dialysate generation flow path **601** can include components required for use in peritoneal dialysate. Upon addition of solutes from the concentrate source **604,** the fluid in the peritoneal dialysate generation flow path **601** can contain all the necessary solutes for peritoneal dialysis. The peritoneal dialysate should reach a level of sterility for peritoneal dialysis, as described. As shown in FIG. 6, the sterilization module can include one or more of a first ultrafilter **607,** a second ultrafilter **609,** and a UV light source **606.**

The generated peritoneal dialysate can be pumped directly to an integrated cycler **610** for immediate infusion into a patient **634.** Alternatively, the dialysate can be pumped to an optional dialysate container **614** as a pre-prepared bolus of solution for storage until ready for use by a patient **634.** Valve **616** can control the movement of fluid to either the dialysate container **614.** Stored dialysate in dialysate container **614** can be pumped as needed to back into the peritoneal dialysate generation flow path **601** by pump **615** through valve **617.** The dialysate container **614** can store enough peritoneal dialysate for a single infusion of peritoneal dialysate into the patient **634,** or enough peritoneal dialysate for multiple or continuous infusions into one or multiple patients.

The generated peritoneal dialysate can be pumped to valve **637.** Valve **637** can control movement of the peritoneal dialysate to any of three options. First, the peritoneal dialysate can be pumped to integrated cycler **610,** second diverted for use with a non-integrated external cycler **639,** or third diverted to a dialysate container **640.** All three options can be performed contemporaneously or selectively. If diverted to the non-integrated external cycler **639,** the peritoneal dialysate can be pumped via valve **638.** Valve **638** can control the movement of the peritoneal dialysate through either a direct connection to an external cycler **639** or to a dialysate container **640.** Alternative valve and pump configurations for performing the same functions are contemplated by the present invention. For example, the direct connection to an external cycler **639** can use any type of connector known in the art. The connectors can be single-use or reusable connectors and should provide for sterile transfer of fluids. The connectors should preferably be closed connectors, to avoid contact between the fluids and the external environment. A non-limiting example of a connector that can be used for a direct connection to an external cycler is the INTACT® connectors provided by Medinstill Development LLC, Delaware, US. The dialysate container **640** can be heated with an optional heater **641** and then used in peritoneal dialysis. The connectors to the dialysate container **640** can be any type of connector known in the art. The connectors can be single use or disposable connectors that provide transfer of sterile fluids. A non-limiting example of connectors that can be used with the described system is the Lynx®-Millipore connectors available from Merck KGaA, Darmstadt, Germany.

The integrated cycler **610** can include a metering pump **619** for metering peritoneal dialysate into the peritoneal cavity of the patient **634.** A heater **618** heats the peritoneal dialysate to a desired temperature prior to infusion into the patient **634.** A pressure regulator **620** ensures the peritoneal dialysate pressure is within a predetermined range safe for infusion into the patient **634.** The metering pump **619** can use any safe pressure for infusing fluid into the patient **634.** Generally, the pump pressures are on average set at ±10.3 kPa or 77.6 mmHg. If there is no fluid flow, the maximum pressure can increase to ±15.2 kPa or 113.8 mmHg for a short period, such as less than 10 seconds. The peritoneal dialysate is infused into the peritoneal cavity of the patient **634** through infusion line **624.** After a dwell period, the peritoneal dialysate is drained from the patient **634** through drain line **623.** Pump **622** provides a driving force for removing the peritoneal dialysate from the patient **634.** An optional waste reservoir **621** can be included to store the used peritoneal dialysate for disposal. Alternatively, the drain line **623** can be directly connected to a drain for direct disposal. The waste reservoir **621** can be any size, including between around 12 and around 25 L. For patients requiring a higher drainage, a drain manifold can be included for connecting multiple waste reservoirs.

Various sensors positioned in the peritoneal dialysate generation and infusion system ensure that the generated fluid is within predetermined parameters. Flow meter **635** ensures the incoming water is at a correct flow rate, while pressure sensor **636** ensures the incoming water is at an appropriate pressure. Conductivity sensor **625** is used to ensure that the water exiting water purification module **603** has been purified to a level safe for use in peritoneal dialysis. Conductivity sensor **626** ensures the conductivity of the dialysate after the addition of concentrates from concentrate source **604** is within a predetermined range. Refractive index sensor **627** insures that the concentration of the osmotic agents is within a predetermined range. pH sensor **628** ensures the pH of the peritoneal dialysate is within a predetermined range. After passing through the sterilization module including second ultrafilter **609,** pH sensor **629** and conductivity sensor **630** are used to ensure that no changes in the pH or conductivity have occurred during purification or storage of the dialysate in dialysate container **614.** The integrated cycler **610** has flow meter **631,** pressure sensor **632** and temperature sensor **633** to ensure that the dialysate being infused into the patient **634** is within a proper flow rate, pressure, and temperature range.

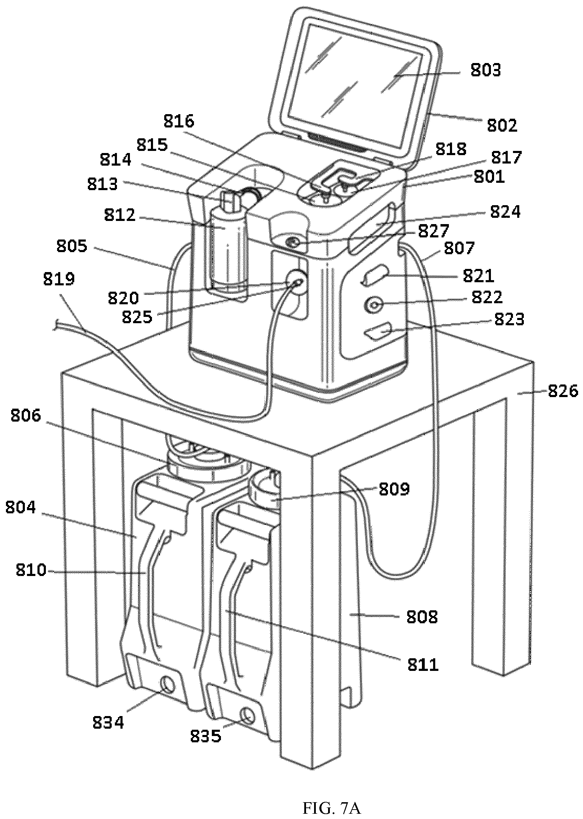

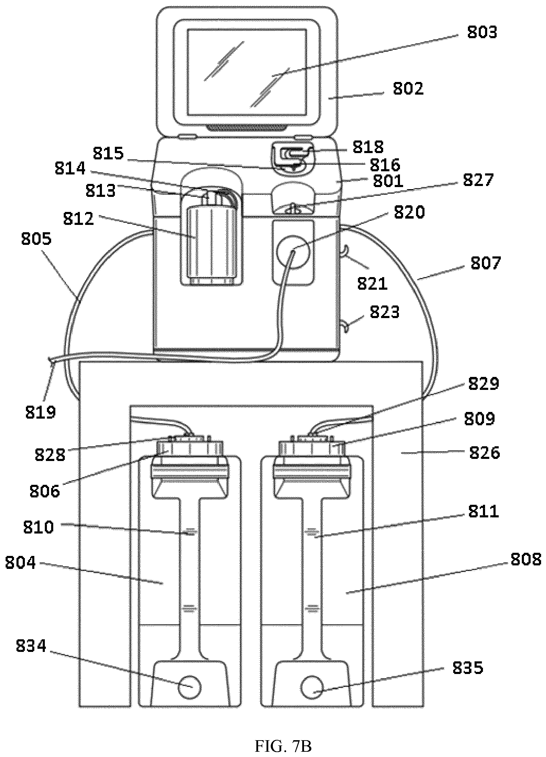

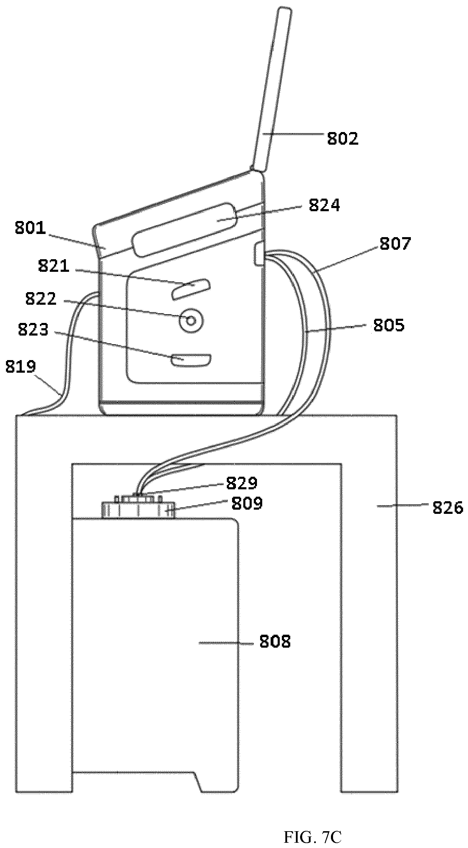

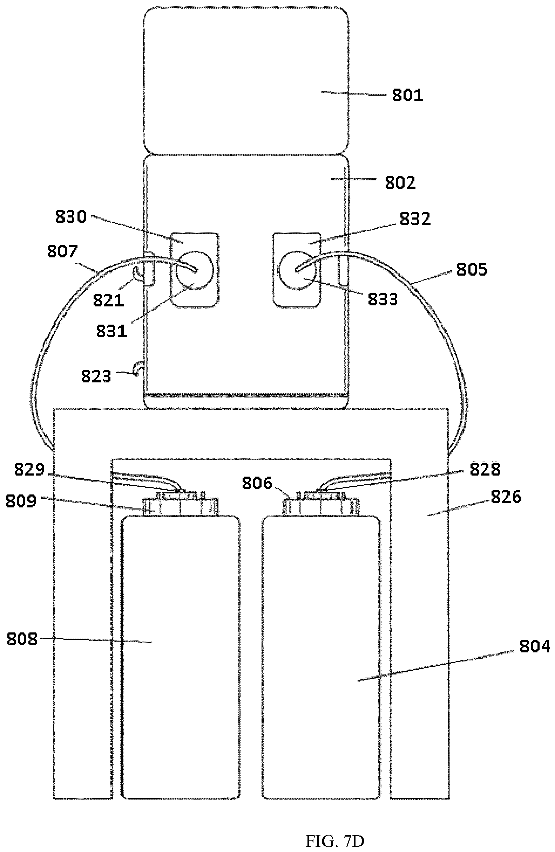

FIG.'s 7A-D illustrate a non-limiting embodiment of the peritoneal dialysate generation system arranged as a peritoneal dialysate generation cabinet **801.** FIG. 7A illustrates a perspective view of the peritoneal dialysate generation cabinet **801,** FIG. 7B illustrates a front view of the peritoneal dialysate generation cabinet **801,** FIG. 7C illustrates a side view of the peritoneal dialysate generation cabinet **801,** and FIG. 7D illustrates a back view of the peritoneal dialysate generation cabinet **801.**

A fluid line **805** can connect a water source **804** to the peritoneal dialysate generation cabinet **801.** The fluid line **805** can enter through a connector **828** in a top **806** of the water source **804.** The fluid line **805** connects to the peritoneal dialysate generation flow path as described with reference to FIG.'s 1 and 5-6 through a back of the peritoneal dialysate generation cabinet **801** through connector **832** having a fitting **833** for holding the fluid line **805,** as illustrated in FIG. 7D. Any of the fluid lines illustrated can be disconnected and removed from the system for cleaning and replacement. A pump (not shown) can provide a driving force for the movement of fluid throughout the peritoneal dialysate generation flow path if required. Water is pumped through the peritoneal dialysate generation cabinet **801** to a water purification module, shown as sorbent cartridge **812** in FIG.'s 7A-B. The water can enter the sorbent cartridge **812** through tubing (not shown) connected to the bottom of the sorbent cartridge **812** within the peritoneal dialysate generation cabinet **801.** The water exits the sorbent cartridge **812** through connector **813** and tubing **814.** An osmotic agent from osmotic agent source **815** and an ion concentrate from an ion concentrate source **817** are added to the fluid as described to generate non-sterilized peritoneal dialysate. The osmotic agent concentrate is added to the fluid through paddle connector **816.** The ion concentrate is added to the fluid through paddle connector **818.** A concentrate pump (not shown) can provide a driving force to move fluid from the concentrate sources into the peritoneal dialysate generation flow path inside of the peritoneal dialysate generation cabinet **801.** As described, the system can use a single ion concentrate source in place of the two sources shown in FIG.'s 7A-B, or more than two concentrate sources. The generated peritoneal dialysate can then be pumped through a sterilization module (not shown), such as an ultrafilter. A second ultrafilter and/or a UV light source can also be included. An integrated cycler (not shown in FIG.'s 7A-D) can then pump the dialysate into infusion line **819** through connector **820** and into the patient. Fitting **825** allows the infusion line **819** to be removed from the system for cleaning or replacement. Waste fluids can be pumped out of the system through waste line **807,** which connects to the peritoneal dialysate generation cabinet **801** through connector **830** having fitting **831.** A separate waste line for removing used dialysate from the patient (not shown in FIG.'s 7A-D) can also connect to the peritoneal dialysate generation cabinet **801** and connect to waste line **807.** The waste line **807** enters waste container **808** through a connector **829** in the top **809** of the waste container **808.** Handles **810** and **811** can be included on water source **804** and waste container **808** for easy movement and storage. Although the peritoneal dialysate generation cabinet **801** is illustrated on top of table **826** in FIG.'s 7A-D, the peritoneal dialysate generation cabinet **801** can be used on any stable flat surface.

As described, the peritoneal dialysate generation flow path can include various sensors for detection of conductivity, pH, refractive index, or other dialysate parameters. The sensors can be included either inside or outside of the body of the peritoneal dialysate generation cabinet **801.** The fluid lines and valves connecting the components of the peritoneal dialysate generation flow path can likewise be positioned inside of the cabinet body. As described, a top of the peritoneal dialysate generation cabinet **801** can have a graphical user interface **802** including screen **803.** Messages from the control system to the user, or from the user to the control system, can be generated and read through the graphical user interface **802.** The user can direct the generation of peritoneal dialysate through the graphical user interface **802,** and can receive messages from the system through screen **803.** The system can generate alerts to the user, including any problems detected by any of the sensors, as well as the progress of peritoneal dialysate generation. A handle **824** can be included for opening the peritoneal dialysate generation cabinet **801** to allow access to components on the inside of the cabinet. Handles **821** and **823** can be included to hold the fluid lines and power cord when not in use.

Disinfection connector **822** illustrated in FIG.'s 7A and 7C can be included for disinfection of the waste line **807.** During disinfection, the waste line **807** can be disconnected from waste container **808** and connected to disinfection connector **822.** Disinfectant solution from a disinfectant source (not shown in FIG.'s 7A-D) can then be circulated through the waste line **807** to disinfect the waste line **807.** Disinfection connector **827** can be included for disinfection of fluid line **805.** Fluid line **805** can be connected to disinfection connector **822** and disinfection solution can be circulated through the fluid line **805.** Drain **834** on water source **804** and drain **835** on waste container **808,** allow the water source **804** and waste container **808** to be drained without inverting the containers.

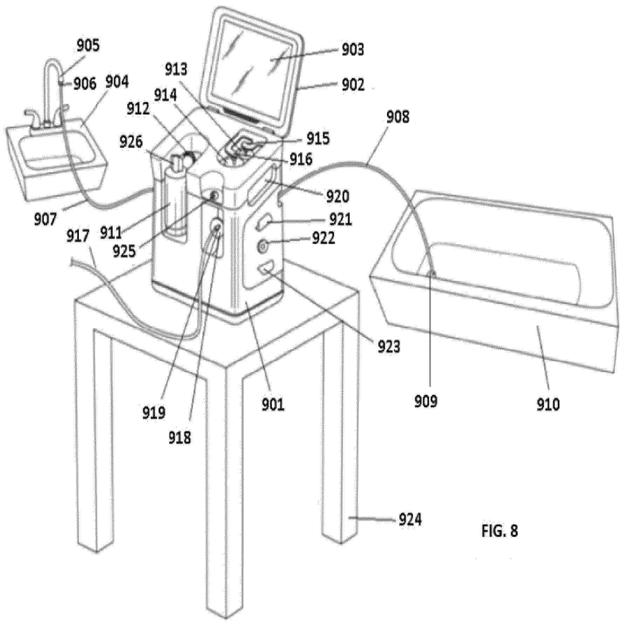

FIG. 8 illustrates a peritoneal dialysate generation cabinet **901** using a non-purified water source, faucet **905** in sink **904.** Although illustrated as faucet **905** and sink **904,** one of ordinary skill in the art will understand that any water source can be used. The ability to use municipal or other non-purified sources of water allow the peritoneal dialysate generation system to work at a patient's home without the need to store large amounts of purified water or dialysate. Fitting **906** connects the water line **907** to the faucet **905** or other water source, allowing the water line **907** to be connected or disconnected as necessary. A pump (not shown) can provide a driving force for the movement of fluid throughout the peritoneal dialysate generation flow path as described with respect to FIG.'s 1 and 5-6. The water is pumped through the peritoneal dialysate generation cabinet **901** to a water purification module, shown as sorbent cartridge **911** in FIG. 8. The water enters the sorbent cartridge **911** through tubing (not shown) connected to the bottom of the sorbent cartridge **911** within the peritoneal dialysate generation cabinet **901.** The water exits the sorbent cartridge **911** through connector **926** and tubing **912.** An osmotic agent from osmotic agent source **913** and an ion concentrate from an ion concentrate source **914** are added to the fluid as described to generate non-sterilized peritoneal dialysate. The osmotic agent concentrate is added to the fluid through paddle connector **916.** The ion concentrate is added to the fluid through paddle connector **915.** A concentrate pump (not shown) can provide a driving force to move fluid from the concentrate sources into the peritoneal dialysate generation flow path inside of the peritoneal dialysate generation cabinet **901.** As described, the system can use a single ion concentrate source in place of the two sources shown in FIG. 8, or more than two concentrate sources. The generated peritoneal dialysate can then be pumped through a sterilization module (not shown), such as an ultrafilter. A second ultrafilter and/or a UV light source can also be included. An integrated cycler (not shown in FIG. 8) can then pump the dialysate into infusion line **917** through connector **918** and into the patient. Fitting **919** allows the infusion line **917** to be removed from the system for cleaning or replacement. Waste fluids can be pumped out of the system through waste line **908,** which can connect to a drain **909** shown in bathtub **910.** A separate drain line (not shown) from the patient can be included to move used dialysate into the drain **909.** Although shown as a bathtub drain **909** in FIG. 8, the waste fluids can be conveyed to any type of drain, or alternatively to a waste container as illustrated in FIG.'s 7A-D. Although the peritoneal dialysate generation cabinet **901** is illustrated on top of table **924** in FIG. 8, the peritoneal dialysate generation cabinet **901** can be used on any stable flat surface. In certain embodiments, the peritoneal dialysate generation cabinet **901** and the patient can be in the same room as the water source and drain **909.** Alternatively, the patient and/or peritoneal dialysate generation cabinet **901** can be in a separate room, with tubing long enough to reach patient. For longer distances, the tubing should be strong enough to withstand the pressures necessary in pumping fluid over longer distances.

As described, a top of the peritoneal dialysate generation cabinet **901** can have a graphical user interface **902** including screen **903.** Messages from the control system to the user, or from the user to the control system, can be generated and read through the graphical user interface **902.** The user can direct the generation of peritoneal dialysate through the graphical user interface **902,** and can receive messages from the system through screen **903.** The system can generate alerts to the user, including any problems detected by any of the sensors, as well as the progress of peritoneal dialysate generation. A handle **920** can be included for opening the peritoneal dialysate generation cabinet **901** to allow access to components on the inside of the cabinet. Handles **921** and **923** can be included to hold the fluid lines and power cord when not in use.

Disinfection connector **922** can be included for disinfection of the waste line **908.** During disinfection, the waste line **908** can be disconnected from the drain **909** and connected to disinfection connector **922.** Disinfectant solution from a disinfectant source (not shown in FIG. 8) can then be circulated through the waste line **908** to disinfect the waste line **908.** Disinfection connector **925** can be included for disinfection of water line **907.** The water line **907** can be disconnected from faucet **905** and connected to disinfection connector **925.** Disinfectant solution can be circulated through the water line **907** for disinfection.

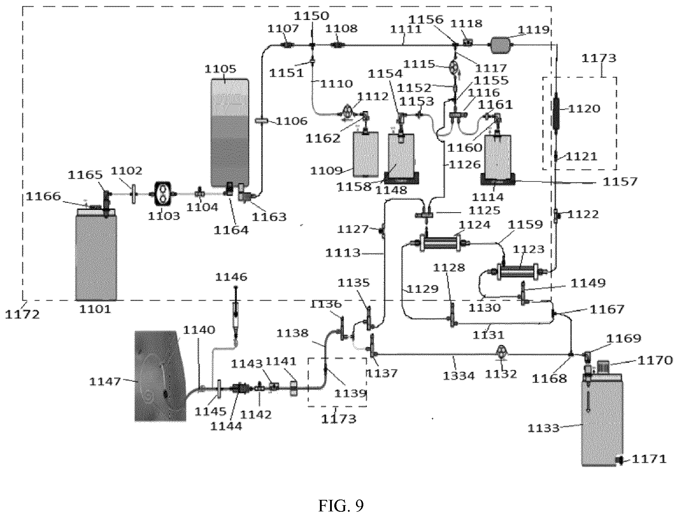

FIG. 9 illustrates an alternative non-limiting embodiment of a peritoneal dialysate generation flow path **1111.** Water from water source **1101** can be pumped into the peritoneal dialysate generation flow path **1111** by system pump **1103** through connector **1165.** Although shown with screw top **1166** in FIG. 9, any method can be used with the water source **1101** to fill and drain the water source **1101.** The water can be pumped through filter **1102** to remove any particulate matter from the water prior to entering the peritoneal dialysate generation flow path **1111.** Alternatively, a dedicated water source, such as a tap or a municipal water source, can be used in place of water source **1101.** Pressure sensor **1104** measures the pressure upstream of sorbent cartridge **1105.** In certain embodiments, an alternative water purification module can be used in place of sorbent cartridge **1105,** including a reverse osmosis module, a nanofilter, a combination of ion and anion exchange materials, activated carbon, silica, or silica based columns. The shading in sorbent cartridge **1105** shows varying layers of sorbent material. However, any order of sorbent material layers can be used, or the sorbent materials can be intermixed. In FIG. 9, the sorbent cartridge **1105** has a fluid inlet **1164** and fluid outlet **1163** in a base of the sorbent cartridge **1105.** In certain embodiments, the fluid inlet **1164** and fluid outlet **1163** can instead be on opposite sides of the sorbent cartridge **1105.** A filter **1106** can remove particulate matter in the fluid exiting sorbent cartridge **1105.**

A first conductivity sensor **1107** can measure the conductivity of the fluid exiting sorbent cartridge **1105.** One or more infusates can be added from ion concentrate source **1109** through connector **1162** to infusate line **1110** by concentrate pump **1112** to the peritoneal dialysate generation flow path **1111** at T-junction **1150.** Filter **1151** can remove any particulate matter from the infusate concentrate prior to reaching the peritoneal dialysate generation flow path **1111.** Alternatively, a valve can be used in place of T-junction **1150.** A secondary conductivity sensor **1108** can measure the conductivity of the fluid after addition of the infusates to ensure proper concentrations of each infusate. As described, the system can include any number of infusate sources, each with the same or separate infusate pumps and infusate lines. A fluid having a specific known concentration of solutes will have a specific conductivity. As such, a control system in communication with secondary conductivity sensor **1108** can measure the conductivity of the fluid with secondary conductivity sensor **1108** to ensure the conductivity is within a predetermined range of a patient dialysate prescription. The control system can also adjust an ion concentrate flow rate by adjusting the pump rate of concentrate pump **1112** based on data received from secondary conductivity sensor **1108.** If the conductivity measured by secondary conductivity sensor **1108** is below a predetermined range from the dialysate prescription, the control system can increase the ion concentrate flow rate. If the conductivity measured by secondary conductivity sensor **1108** is above a predetermined range from the dialysate prescription, the control system can decrease the ion concentrate flow rate.

A secondary concentrate pump **1115** forming part of a secondary infusate line **1117** can add an osmotic agent to the peritoneal dialysate generation flow path **1111** through secondary infusate line **1117** at T-junction **1156.** Although shown as a single secondary infusate line **1117** in FIG. 9, one of skill in the art will understand that any number of secondary infusate lines can be used to connect separate osmotic agent sources to the peritoneal dialysate generation flow path **1111.** A secondary composition sensor **1152** can measuring the osmotic agent concentration of the fluid in secondary infusate line **1117.** A control system in communication with a secondary composition sensor **1152** can use the osmotic agent concentration in secondary infusate line **1117** in setting an osmotic agent flow rate based on a dialysate prescription. The final osmotic agent concentration in the dialysate will be a function of the osmotic agent concentration in secondary infusate line **1117** and the relative rates of the dialysate flow through peritoneal dialysate generation flow path **1111** and secondary infusate line **1117.** As illustrated in FIG. 9, the system can have multiple osmotic agent sources, including dextrose source **1148** fluidly connected to osmotic agent line through connector **1154** and icodextrin source **1114** fluidly connected to osmotic agent line through connector **1160.** The user or control system can select the appropriate osmotic agent to use based on the needs of the patient, as described. Filter **1153** can remove particulate matter from fluid exiting dextrose source **1148** and filter **1161** can remove particulate matter form fluid exiting icodextrin source **1114.** Alternative osmotic agent sources, including an amino acid source or a glucose source, can be used in place of, or in addition to, the dextrose source **1148** and icodextrin source **1114,** allowing customization of the osmotic agents used. Valve **1116** controls the source from which the osmotic agent is obtained. Alternatively, multiple osmotic agent lines and osmotic agent pumps can be used. A flow meter **1118** measures the flow rate of fluid through the peritoneal dialysate generation flow path **1111.** A composition sensor **1119** can measure the concentrations of the osmotic agents in the fluid, as well as the infusates. The composition sensor can include a single sensor, or multiple sensors measuring separate fluid parameters. The composition sensors **1152** and **1119** can include a refractive index sensor, an enzyme-based sensor, and/or a pulsed amperometric detection sensor. The composition sensors **1152** and **1119** can also include conductivity sensors, pH sensors, and/or flow meters. Using composition sensor **1119** and secondary conductivity sensor **1108,** the control system can determine the concentrations of the ions and osmotic agents in peritoneal dialysate generation flow path **1111.** If the osmotic agent concentration or ion concentrations outside of a predetermined range from the dialysate prescription, the system can generate an alert and/or stop treatment. The system can also adjust the ion concentrate flow rate and/or osmotic agent flow rate to bring the ion concentration and osmotic agent concentrations to within the predetermined range of the dialysate prescription.

Heater **1120** heats the fluid in the peritoneal dialysate generation flow path **1111** to the patient body temperature. Temperature sensor **1121** measures the temperature of the fluid and can be used to by a control system to control the heater **1120,** heating the fluid to a temperature of between around 25°C to around 40°C. In a preferred embodiment, the desired temperature can be 37 ± 2 °C or between 36.5 to 37.25°C. A control system can monitor the temperature and shut off flow or generate an alarm if the temperature is outside of the desired range. In certain embodiments, the control system can shut off flow if the temperature is equal to greater than around 41 °C. Pressure sensor **1122** measures the pressure of the fluid prior to entering a dialysate sterilization module.

The dialysate sterilization module can include a first ultrafilter **1123** and a second ultrafilter **1124** fluidly connected by fluid line **1159.** The fluid flows through both ultrafilters to remove any chemical or biological contaminants. Waste fluid can exit the first ultrafilter **1123** through fluid line **1130** and exit the second ultrafilter **1124** through fluid line **1129.** Valves **1149** and **1128** control the movement of fluid between the first ultrafilter **1123** and second ultrafilter **1124** into waste line **1131,** which is fluidly connected to fluid line **1130** at T-junction **1167.** Valves **1149** and **1128** can be used to modulate the fluid movement out of ultrafilters **1123** and **1124** to ensure sufficient pressure for ultrafiltration. If the pressure in ultrafilter **1124** decreases below a necessary value, valve **1128** can be closed, preventing fluid movement from ultrafilter **1123** into fluid line **1130** and increasing the pressure in ultrafilter **1124.** The waste line **1131** is fluidly connected to a waste line **1134** at T-junction **1168** and to waste reservoir **1133** through connector **1169,** or alternatively, to a drain. Although shown with a screw top **1170** and tap **1171,** one of skill in the art will understand that alternative methods for filling and draining waste reservoir **1133** can be used.

Fluid exiting the second ultrafilter **1124** passes through valve **1125.** Valve **1125** can direct the fluid into either fluid line **1113** and an integrated cycler or into fluid line **1126** for addition to the dextrose source **1148** and icodextrin source **1114** via T-junction **1155.** The fluid can be added to dextrose source **1148** and icodextrin source **1114** to dissolve solid icodextrin and solid dextrose prior to generating the peritoneal dialysate.

Fluid line **1113** can include a pressure sensor **1127** to ensure that the fluid pressure is within predetermined limits prior to entering the integrated cycler. Valve **1135** controls the movement of fluid from the sterilization module. Valve **1136** controls the movement of fluid into and out of the integrated cycler through cycler line **1138.**

The cycler line **1138** can include a second temperature sensor **1139** to ensure the proper temperature of the peritoneal dialysate prior to infusion into the patient **1147.** An air detector **1141** is included to detect any air that would otherwise be introduced into the patient **1147.** A bubble trap (not shown) can be included to remove any detected air. A flow meter **1143** measures the flow rate of fluid in the cycler line **1138** and can be used to control the amount of peritoneal dialysate infused into the patient **1147.** A pressure sensor **1142** can be included to ensure the fluid pressure in cycler line **1138** is within predetermined limits for infusion into the patient **1147.** A catheter **1140** can connect to the cycler line **1138** at connection **1144.** In certain embodiments, a heparin syringe **1146** can be included to add heparin or other medication to the peritoneal dialysate. Filter **1145** removes any particulate matter prior to infusion into the patient **1147.**

After a dwell period, the spent peritoneal dialysate can be drained from the patient **1147** through the cycler line **1138.** Drain pump **1132** can provide the driving force for draining the spent peritoneal dialysate. The spent peritoneal dialysate passes through valves **1136** and **1137** and into drain line **1134,** which can fluidly connect to waste reservoir **1133** or to a drain.

As illustrated in FIG. 9, the dialysate preparation system can be fluidly connected to a dialysate preparation system. The dialysate preparation system can include the conductivity sensors **1107** and **1108,** the ion concentrate source **1109,** one or more osmotic agent sources illustrated as icodextrin source **1114** and dextrose source **1148,** infusate lines **1110** and **1117,** and composition sensors **1152** and **1119,** shown as dashed box **1172.** The dialysate generation system can also include a water purification module, illustrated as sorbent cartridge **1105** in FIG. 9, and a sterilization module illustrated as ultrafilters **1123** and **1124** in FIG. 9.

As illustrated in FIG. 9, the secondary infusate line **1117** can be fluidly connected to the second ultrafilter **1124** of the sterilization module by fluid line **1126.** In certain embodiments, a solid source of the osmotic agents and/or infusates can be used. The solid source can be placed in dextrose source **1148** and icodextrin source **1114.** To generate an osmotic agent concentrate, water from water source **1101** can be added to the dextrose source **1148** and icodextrin source **1114,** generating a concentrate of known concentration. To ensure that the icodextrin source **1114** and dextrose source **1148** remain free from chemical or biological contamination, the water can first be passed through the sterilization module, including first ultrafilter **1123** and second ultrafilter **1124** prior to addition to the osmotic agent sources. Water from water source **1101** can be pumped through sorbent cartridge **1105** and peritoneal dialysate generation flow path **1111.** Heater **1120** heats the water and is controlled by a control system based on data received from temperature sensor **1121.** The control system can control heater **1120** to heat the water to a set temperature, which will affect the solubility of the osmotic agents and allow the osmotic agent concentrate to be of known concentration. The water is then pumped through first ultrafilter **1123,** through fluid line **1159** and second ultrafilter **1124.** Valve **1125** can direct the water from the second ultrafilter **1124** through fluid line **1126** and into each of icodextrin source **1114** and dextrose source **1148** via secondary infusate line **1117** to generate osmotic agent concentrates free from contamination. As described, the peritoneal dialysate generation system can include any number of osmotic agent sources, and the second ultrafilter **1124** can be fluidly connected to each osmotic agent source. Although not illustrated in FIG. 9, the second ultrafilter **1124** can also be fluidly connected to the ion concentrate source **1109** to generate an ion concentrate free from chemical or biological contamination. Heater **1120** can heat the water prior to passing through the first ultrafilter **1123** and second ultrafilter **1124.** Using heated water to dissolve the osmotic agents can allow for a faster and more complete dissolution in order to minimize system preparation time before treatment can begin. In certain embodiments, the water can be heated to between around 25°C to around 90°C to minimize dissolution time of the PD fluid components, while also minimizing formation of glucose degradation products. The osmotic agent concentrate can then be mixed with the purified water in the peritoneal dialysate generation flow path **1111** and the temperature can be diluted down by the incoming stream..Vibration plate **1157** can agitate the solution in icodextrin source **1114,** and vibration plate **1158** can agitate the solution in dextrose source **1148** to further speed dissolution of the osmotic agents. A vibration plate or other means of agitating the ion concentrate source can be included. One of skill in the art will understand that alternative means of agitating the ion concentrates can be used, including stirrers or other mixers.

One skilled in the art will understand that various combinations and/or modifications and variations can be made in the described systems and methods depending upon the specific needs for operation. Moreover, features illustrated or described as being part of an aspect of the invention may be used in the aspect of the invention, either alone or in combination, or follow a preferred arrangement of one or more of the described elements.

The invention may be described by reference to the following numbered paragraphs:-
1. A dialysate preparation system for use in peritoneal dialysis, comprising:
   a first fluid line fluidly connected to a water purification module;
   at least one ion concentrate source fluidly connected to the first fluid line through a first infusate line; the first infusate line having a first concentrate pump;
   one or more osmotic agent sources fluidly connected to the first fluid line through one or more secondary infusate lines; the secondary infusate lines comprising a secondary concentrate pump forming part of the one or more secondary infusates lines;
   wherein at least one or more conductivity sensors are positioned in the first fluid line upstream of the first infusate line; at least one or more secondary conductivity sensors are positioned in the first fluid line downstream of the first infusate line and upstream of the secondary infusate lines; and at least one composition sensor positioned in the first fluid line downstream of the one or more secondary infusate lines;
   the first fluid line fluidly connectable to an integrated cycler.
2. The dialysate preparation system of paragraph 1, further comprising at least one secondary composition sensor positioned in the one or more secondary infusate lines.
3. The dialysate preparation system of paragraph 2, further comprising a control system in communication with the composition sensor and secondary composition sensor, the control system measuring an osmotic agent concentration at the composition sensor and secondary composition sensor.
4. The dialysate preparation system of paragraph 3, the control system controlling an osmotic agent flow rate based on the composition sensor and secondary composition sensor.
5. The dialysate preparation system of paragraph 1, further comprising at least one flow meter in the first fluid line.
6. The dialysate preparation system of paragraph 1, wherein at least two osmotic agent sources are fluidly connected to the one or more secondary infusate lines.
7. The dialysate preparation system of paragraph 6, further comprising one or more valves fluidly connecting the at least two osmotic agent sources to the secondary infusate line.
8. The dialysate preparation system of paragraph 1, further comprising a control system in communication with the conductivity sensor and secondary conductivity sensor, the control system controlling an ion concentrate flow rate based on the conductivity sensor and secondary conductivity sensor.
9. The dialysate preparation system of paragraph 1, further comprising at least one pH sensor in the first fluid line.
10. The dialysate preparation system of paragraph 2, wherein the composition sensor and/or secondary composition sensor are selected from the group consisting of a refractive index sensor, an enzyme-based sensor, and a pulsed amperometric detection sensor.
11. The dialysate preparation system of paragraph 1, further comprising a second fluid line fluidly connecting the secondary infusate line to a sterilization module.
12. A method of generating a peritoneal dialysate, comprising:
   a) pumping water from a water source through a water purification module into a first fluid line;
   b) measuring a first conductivity of fluid in the first fluid line;
   c) pumping an ion concentrate from at least one ion concentrate source through a first infusate line into the first fluid line;
   d) measuring a second conductivity of the fluid in the first fluid line downstream of the first infusate line;
   e) pumping an osmotic agent concentrate from an osmotic agent source through a second infusate line into the first fluid line;
   f) measuring a first osmotic agent concentration in the first fluid line downstream of the second infusate line.
13. The method of paragraph 12, further comprising the step of measuring a second osmotic agent concentration in the second infusate line.
14. The method of paragraph 12, further comprising the steps of pumping fluid from the first fluid line into a sterilization module and pumping the fluid from the sterilization module into an integrated cycler.
15. The method of paragraph 12, further comprising receiving a dialysate prescription; and setting an ion concentrate flow rate and an osmotic agent flow rate based on the dialysate prescription.
16. The method of paragraph 13 wherein the step of setting an ion concentrate flow rate and an osmotic agent flow rate is performed by a control system in communication with a first concentrate pump in the first infusate line and a second concentrate pump in the second infusate line.
17. The method of paragraph 16, wherein the control system sets the osmotic agent flow rate based on the first osmotic agent concentration and the dialysate prescription.
18. The method of paragraph 15, further comprising the step of generating an alert if the first osmotic agent concentration and/or the second conductivity are outside of a predetermined range from the dialysate prescription.
19. The method of paragraph 12, further comprising either or both of:
   a) generating the ion concentrate by pumping purified water from a sterilization module into the ion concentrate source; and/or
   b) generating the osmotic agent concentrate by pumping purified water from the sterilization module into the osmotic agent source.
20. The method of paragraph 19, wherein either or both of:
   a) the step of generating the ion concentrate further comprises agitating the ion concentrate after pumping the purified water into the ion concentrate source, heating the purified water prior to pumping the purified water into the ion concentrate source, or combinations thereof; and/or
   b) the step of generating the osmotic agent concentrate further comprises agitating the osmotic agent concentrate after pumping the purified water into the osmotic agent source, heating the purified water prior to pumping the purified water into the osmotic agent source, or combinations thereof.

## Claims

1. A dialysate preparation system for use in peritoneal dialysis, comprising:
a first fluid line fluidly connected to a water purification module;
at least one ion concentrate source fluidly connected to the first fluid line through a first infusate line; the first infusate line having a first concentrate pump;
one or more osmotic agent sources fluidly connected to the first fluid line through one or more secondary infusate lines; the secondary infusate lines comprising a secondary concentrate pump forming part of the one or more secondary infusates lines;
wherein at least one or more conductivity sensors are positioned in the first fluid line upstream of the first infusate line; at least one or more secondary conductivity sensors are positioned in the first fluid line downstream of the first infusate line and upstream of the secondary infusate lines; and at least one composition sensor positioned in the first fluid line downstream of the one or more secondary infusate lines;
the first fluid line fluidly connectable to an integrated cycler.

2. The dialysate preparation system of claim 1, further comprising at least one secondary composition sensor positioned in the one or more secondary infusate lines, preferably wherein the composition sensor and/or secondary composition sensor are selected from the group consisting of a refractive index sensor, an enzyme-based sensor, and a pulsed amperometric detection sensor.

3. The dialysate preparation system of claim 2, further comprising a control system in communication with the composition sensor and secondary composition sensor, the control system measuring an osmotic agent concentration at the composition sensor and secondary composition sensor.

4. The dialysate preparation system of claim 3, the control system controlling an osmotic agent flow rate based on the composition sensor and secondary composition sensor.

5. The dialysate preparation system of any preceding claim, further comprising at least one flow meter in the first fluid line.

6. The dialysate preparation system of any preceding claim, wherein at least two osmotic agent sources are fluidly connected to the one or more secondary infusate lines, preferably further comprising one or more valves fluidly connecting the at least two osmotic agent sources to the secondary infusate line.

7. The dialysate preparation system of any preceding claim, further comprising a control system in communication with the conductivity sensor and secondary conductivity sensor, the control system controlling an ion concentrate flow rate based on the conductivity sensor and secondary conductivity sensor.

8. The dialysate preparation system of any preceding claim, further comprising at least one pH sensor in the first fluid line.

9. The dialysate preparation system of any preceding claim, further comprising a second fluid line fluidly connecting the secondary infusate line to a sterilization module.

10. A method of generating a peritoneal dialysate, comprising:
a) pumping water from a water source through a water purification module into a first fluid line;
b) measuring a first conductivity of fluid in the first fluid line;
c) pumping an ion concentrate from at least one ion concentrate source through a first infusate line into the first fluid line;
d) measuring a second conductivity of the fluid in the first fluid line downstream of the first infusate line;
e) pumping an osmotic agent concentrate from an osmotic agent source through a second infusate line into the first fluid line;
f) measuring a first osmotic agent concentration in the first fluid line downstream of the second infusate line.

11. The method of claim 10, further comprising the step of measuring a second osmotic agent concentration in the second infusate line, preferably wherein the step of setting an ion concentrate flow rate and an osmotic agent flow rate is performed by a control system in communication with a first concentrate pump in the first infusate line and a second concentrate pump in the second infusate line.

12. The method of claim 10 or claim 11, further comprising the steps of pumping fluid from the first fluid line into a sterilization module and pumping the fluid from the sterilization module into an integrated cycler.

13. The method of any of claims 10 to 12, further comprising receiving a dialysate prescription; and setting an ion concentrate flow rate and an osmotic agent flow rate based on the dialysate prescription, preferably wherein the control system sets the osmotic agent flow rate based on the first osmotic agent concentration and the dialysate prescription, and preferably further comprising the step of generating an alert if the first osmotic agent concentration and/or the second conductivity are outside of a predetermined range from the dialysate prescription..

14. The method of claim 11, further comprising either or both of:
a) generating the ion concentrate by pumping purified water from a sterilization module into the ion concentrate source; and/or
b) generating the osmotic agent concentrate by pumping purified water from the sterilization module into the osmotic agent source.

15. The method of claim 14, wherein either or both of:
a) the step of generating the ion concentrate further comprises agitating the ion concentrate after pumping the purified water into the ion concentrate source, heating the purified water prior to pumping the purified water into the ion concentrate source, or combinations thereof; and/or
b) the step of generating the osmotic agent concentrate further comprises agitating the osmotic agent concentrate after pumping the purified water into the osmotic agent source, heating the purified water prior to pumping the purified water into the osmotic agent source, or combinations thereof.
